# EUROPEAN PATENT SPECIFICATION

(11) **EP 3 509 619 B1**
(45) Date of publication and mention of the grant of the patent: **26.06.2024**
(21) Application number: 17780219.6
(22) Date of filing: 06.09.2017
(51) Int. Cl.: A61K 38/08, A61K 38/10, C07K 7/06, C07K 7/08

(54) **PEPTIDES AND PEPTIDE CONJUGATES FOR TREATING MENTAL DISORDERS**
PEPTIDE UND PEPTIDKONJUGATE ZUR BEHANDLUNG VON PSYCHISCHEN ERKRANKUNGEN
PEPTIDES ET CONJUGUÉS PEPTIDIQUES POUR LE TRAITEMENT DE TROUBLES MENTAUX

(30) Priority: 07.09.2016 US 201662384333 P
(43) Date of publication of application: 17.07.2019
(73) Proprietor: Kyoto University, Kyoto-shi, Kyoto 606-8501 (JP); Kazusa DNA Research Institute, Kisarazu-shi, Chiba 292-0818 (JP)
(72) Inventor: OHINATA, Kousaku, Kyoto-shi Kyoto 606-8501 (JP); MORI, Yukiha, Kyoto-shi Kyoto 606-8501 (JP); SUZUKI, Hideyuki, Kisarazu-shi Chiba 292-0818 (JP)
(74) Representative: Hoffmann Eitle
(86) International application number: PCT/JP2017/032079
(87) International publication number: WO 2018/047852

(56) References cited:
- EP-A1- 2 557 088
- WO-A1-2013/143026
- WO-A1-2016/140277
- WO-A2-03/072599
- US-A1- 2010 210 564
- OTA AMI ET AL: "Rational identification of a novel soy-derived anxiolytic-like undecapeptide acting via gut-brain axis after oral administration.", NEUROCHEMISTRY INTERNATIONAL MAY 2017, vol. 105, May 2017 (2017-05), pages 51-57, XP002775557, ISSN: 1872-9754
- MASAAKI YOSHIKAWA: "Bioactive peptides derived from natural proteins with respect to diversity of their receptors and physiological effects", PEPTIDES, vol. 72, 1 October 2015 (2015-10-01), pages 208-225, XP055424806, AMSTERDAM, NL ISSN: 0196-9781, DOI: 10.1016/j.peptides.2015.07.013
- TAKASHI NISHI ET AL: "The Soybean Beta-Conglycin Beta-51-63 Fragment Suppresses Appetite by Stimulating Cholecystokinin Release in Rats", THE JOURNAL OF NUTRITION, AMERICAN SOCIETY FOR NUTRITION, US, 1 January 2003 (2003-01-01), pages 2537-2542, XP002577476, ISSN: 0022-3166
- YUKIHA MORI ET AL: "Characterization of soy-deprestatin, a novel orally-active decapeptide that exerts antidepressant-like effects via gut-brain communication", THE FASEB JOURNAL, 27 September 2017 (2017-09-27), XP055424793, US ISSN: 0892-6638, DOI: 10.1096/fj.201700333RR
- AMI OTA ET AL: "A novel bioactive peptide derived from soy beta-conglycinin exhibits anxiolytic activity in mice", PEPTIDE SCIENCE: PROCEEDINGS OF THE 50TH JAPANESE PEPTIDE SYMPOSIUM, 6TH - 8TH NOVEMBER 2013; OSAKA, JAPAN, 6 November 2013 (2013-11-06), pages 255-256, XP009520477, ISSN: 1344-7661

## Description

### Technical Field

### 1. Cross-reference to related applications

This application claims the priority benefit of U.S. provisional application no. 62/384,333 filed September 7, 2016.

### Background Art

### 2. BACKGROUND

It is estimated that 9.5% of the adult population of the United States suffers from a mood disorder (Kessler et al., 2005, Arch Gen Psychiatry 62(6):617-627). Only about one-half of those suffering from a mood disorder receive treatment, and less than 40% of those receiving treatment receive minimally adequate treatment (Wang et al., 2005, Arch Gen Psychiatry 62(6):629-640).

It is also estimated that 18.1% of the adult population of the United States suffers from an anxiety disorder (Kessler et al., 2005, Arch Gen Psychiatry 62(6):617-627). Less than 40% of those suffering from an anxiety disorder receive treatment, and less than 35% of those receiving treatment receive minimally adequate treatment (Wang et al., 2005, Arch Gen Psychiatry 62(6):629-640).

Disorders of diminished motivation occur frequently in individuals with traumatic brain injury, with estimates for the frequency varying from 5% to 67% (Marin and Wilkosz, 2005, J Head Trauma Rehabil 20(4):377-388). Diminished motivation is also often found in individuals suffering from depression, Parkinson's disease, Alzheimer's disease, and schizophrenia, as well as individuals who have suffered a stroke, and even in healthy individuals, particularly the elderly (Bonnelle et al., 2015, Journal of Physiology 109:16-26).

US 2010/210564 A1 relates to a pharmaceutical or a foodstuff including as an active ingredient a peptide containing Tyr (Y), Phe (F), Trp (W), or His (H) and a hydrophobic amino acid adjacent thereto, or an analog thereof.

WO 2016/140277 A1 relates to a peptide comprising any one of (i) an amino acid sequence LSSTQAQQSY, (ii) an amino acid sequence LSSTQAQQSW and (iii) an amino acid sequence LSSTQAQQSF.

Ota Ami et al. relates to the rational identification of a novel soy-derived anxiolytic-like undecapeptide acting via gut-brain axis after oral administration.

M Yoshikawa et al. relates to bioactive peptides derived from natural proteins with respect to diversity of their receptors and physiological effects.

EP 2 557 088 A1 relates to a pharmaceutical or a food that comprises, as an active ingredient, at least one peptide selected from the group consisting of Val-Tyr-Leu-Pro-Arg, Tyr-Leu-Pro-Arg, and Leu-Pro-Arg, or an analog thereof.

T Nishi et al. relates to the Soybean Beta-Conglycin Beta-51-63 Fragment Suppresses Appetite by Stimulating Cholecystokinin Release in Rats.

Y Mori et al. relates to the characterization of soy-deprestatin, a novel orally-active decapeptide that exerts antidepressant-like effects via gut-brain communication.

Ami Ota et al. relates to the rational identification of a novel soy-derived anxiolytic-like undecapeptide acting via gut-brain axis after oral administration.

WO 03/072599 A2 relates to the novel chemokine binding peptides capable of modulating the biological activity of chemokines.

WO 2013/143026 A1 relates to antibody libraries, polypeptide libraries and the uses thereof and also methods for producing antibody libraries, methods for identifying an antibody to a target, and methods for identifying a target associated with a condition.

### Solution to Problem

### 3. SUMMARY

The present invention is defined in the claims and provides a pharmaceutical composition comprising a compound that is a peptide or a salt thereof and one or more pharmaceutically acceptable carriers, diluents and/or excipients, wherein the amino acid sequence of the peptide:
a. consists of:
   i. 5 to 10 consecutive amino acids from the amino acid sequence LSSTQAQQSX1 (SEQ ID NO:34), where X1 is Y, W, or F; and
   ii. optionally, an additional aromatic amino acid at the C-terminus of said 5 to 10 consecutive amino acids; and
b. does not consist of the amino acid sequence LSSTQAQQSY (SEQ ID NO:1), LSSTQAQQS (SEQ ID NO:2), SSTQAQQSY (SEQ ID NO:3), LSSTQ (SEQ ID NO:4), AQQSY (SEQ ID NO:5), LSSTQAQQSW (SEQ ID NO:6), or LSSTQAQQSF (SEQ ID NO:7).

The present invention also provides a pharmaceutical composition comprising a compound that is a conjugate or a salt thereof, the conjugate comprising:
a. a peptide moiety whose amino acid sequence:
   i. consists of
   (I) 5 to 10 consecutive amino acids from the amino acid sequence LSSTQAQQSX1 (SEQ ID NO:34), where X1 is Y, W, or F; and
   (II) optionally, an additional aromatic amino acid at the C-terminus of said 5 to 10 consecutive amino acids; and
   iii. does not consist of the amino acid sequence LSSTQAQQSY (SEQ ID NO:1), LSSTQAQQSW (SEQ ID NO:6), or LSSTQAQQSF (SEQ ID NO:7);
   attached to
b. one or more conjugate moieties.

Further aspects of the invention are also defined in the claims.

Any references in the description to methods of treatment refer to the compounds, pharmaceutical compositions and medicaments of the present invention for use in a method of treatment of the human (or animal) body by therapy.

PCT international application no. PCT/JP2016/056453 describes 10 amino acid peptides derived from the soybean storage protein β-conglycinin having amino acid sequences LSSTQAQQSY (SEQ ID NO:1), LSSTQAQQSW (SEQ ID NO: 6) and LSSTQAQQSF (SEQ ID NO:7), and their usefulness for treating conditions such as depression and decreased motivation.

The disclosure provides peptides and salts thereof that are 5 to 11 amino acids in length and have an amino acid sequence comprising at least 5 consecutive amino acids from the amino acid sequence LSSTQAQQSX₁ (SEQ ID NO:34), where X₁ is Y, W, of F, as defined in the claims. In certain embodiments, in the claims the peptides are 5-8, 6-10 or 7-11 amino acids in length. Exemplary peptides and salts thereof are described in Section 5.1.

The disclosure provides peptide conjugates and salts thereof comprising a peptide moiety attached to one or more (e.g., one, two or three) conjugate moieties, as defined in the claims. In certain embodiments, the peptide moieties are 5-8, 6-10 or 7-11 amino acids in length. Exemplary peptide conjugates and salts thereof are described in Section 5.2.

The disclosure also provides pharmaceutical compositions and food products comprising a compound of the disclosure. Exemplary pharmaceutical compositions are described in Section 5.3 and exemplary food products (e.g., dietary supplements and functional foods) are described in Section 5.4

The disclosure also provides methods for treating or preventing a mood disorder, an anxiety disorder, or a disorder of diminished motivation using the compounds, pharmaceutical compositions, and food products described herein. Exemplary methods of treating or preventing mood disorders, anxiety disorders, and disorders of diminished motivation are described in Section 5.5.

### Brief Description of Drawings

### 4. BRIEF DESCRIPTION OF THE FIGURES

[fig.1]FIG. 1 shows the results of a tail suspension test in mice orally administered the peptide LSSTQAQQSY (SEQ ID NO:1).
[fig.2]FIG. 2 shows the results of a forced swimming test in mice orally administered the peptide LSSTQAQQSY (SEQ ID NO:1).
[fig.3]FIGS. 3A-3B show the results of a tail suspension test in mice orally administered the peptide LSSTQAQQSY (SEQ ID NO:1), LSSTQAQQS (SEQ ID NO:2), or SSTQAQQSY (SEQ ID NO:3) (Fig. 3A), or LSSTQAQQSY (SEQ ID NO:1), LSSTQ (SEQ ID NO:4), or AQQSY (SEQ ID NO:5) (Fig. 3B).
[fig.4]FIGS. 4A-C show the results of an elevated plus maze test in mice orally administered the peptide LSSTQAQQSY (SEQ ID NO:1). Fig. 4A shows the percentage of time the mice spent in the open arms of the maze. Fig. 4B shows the percentage of visits to arms of the maze which were made to the open arms. Fig. 4C shows the total number of visits to both the open and closed arms of the maze.
[fig.5]FIGS. 5A-5C show the results of an open field test in mice orally administered the peptide LSSTQAQQSY (SEQ ID NO:1). Fig. 5A shows the percentage of time the mice spent in the 12 cm circle at the center of the test chamber. Fig. 5B: shows the number of visits to the 12 cm circle. Fig. 5C shows the locomotor activity of the mice.
[fig.6]FIG. 6 shows the results of a tail suspension test in mice orally administered the peptide LSSTQAQQSY (SEQ ID NO:1), LSSTQAQQSW (SEQ ID NO:6), or LSSTQAQQSF (SEQ ID NO:7), or the C-terminal amidated peptide conjugate LSSTQAQQSY-NH₂ (SEQ ID NO:36).
[fig.7]FIG. 7 shows the results of a tail suspension test in vagotomized mice and sham operated mice orally administered the peptide LSSTQAQQSY (SEQ ID NO:1).
[fig.8]FIG. 8 shows the results of a tail suspension test in mice orally administered the peptide LSSTQAQQSY (SEQ ID NO:1) alone or in combination with an antagonist of serotonin 5-HT_{1A}, dopamine D₁, or GABA_{A} receptors.
[fig.9]FIG. 9 shows the results of a tail suspension test in mice orally administered the peptides LSSTQAQQ (SEQ ID NO:27), SSTQAQQS (SEQ ID NO:28), and STQAQQSY (SEQ ID NO:29).
[fig.10]FIG. 10 shows the results of a tail suspension test in mice orally administered the peptides LSSTQAQQSYW (SEQ ID NO:37), WLSSTQAQQSY (SEQ ID NO:38), WLSSTQAQQSYW (SEQ ID NO:39), WLSSTQ (SEQ ID NO:40), and AQQSYW (SEQ ID NO:41).
[fig.11]FIG. 11 shows the results of a tail suspension test in mice orally administered the peptides ESFFLSSTQAQQSY (SEQ ID NO:42), LSSTQAQQSYLQGF (SEQ ID NO:43), and FFLSSTQAQQSYLQ (SEQ ID NO:44).
[fig.12]FIG. 12 shows the results of a tail suspension test in mice orally administered the peptides SSTQAQQS (SEQ ID NO:28), LSSTQAQQSYW (SEQ ID NO:37) and WLSSTQ (SEQ ID NO:40).

### Description of Embodiments

### 5. DETAILED DESCRIPTION

### 5.1. Peptides

The disclosure provides a pharmaceutical composition according to claim 1 It should be understood that when an embodiment described herein refers to a "peptide," the embodiment encompasses the peptide per se as well as salts of the peptide even though the embodiment may not explicitly recite the expression "or salt thereof' or similar, unless required otherwise by context.

The peptides of the disclosure are 5 to 11 amino acids (i.e., 5, 6, 7, 8, 9, 10 or 11 amino acids) in length. In some embodiments, a peptide of the disclosure is 5 amino acids in length. In other embodiments, a peptide of the disclosure is 6 amino acids in length. In other embodiments, a peptide of the disclosure is 7 amino acids in length. In other embodiments, a peptide of the disclosure is 8 amino acids in length. In other embodiments, a peptide of the disclosure is 9 amino acids in length. In other embodiments, a peptide of the disclosure is 10 amino acids in length. In other embodiments, a peptide of the disclosure is 11 amino acids in length.

In some embodiments, the peptide comprises or consists of the amino acid sequence SSTQA (SEQ ID NO:8). In other embodiments, the peptide comprises or consists of the amino acid sequence STQAQ (SEQ ID NO:9). In other embodiments, the peptide comprises or consists of the amino acid sequence TQAQQ (SEQ ID NO:10). In other embodiments, the peptide comprises or consists of the amino acid sequence QAQQS (SEQ ID NO:11). In other embodiments, the peptide comprises or consists of the amino acid sequence AQQSW (SEQ ID NO:12). In other embodiments, the peptide comprises or consists of the amino acid sequence or AQQSF (SEQ ID NO:13).

In some embodiments, the peptide comprises or consists of the amino acid sequence LSSTQA (SEQ ID NO:14). In other embodiments, the peptide comprises or consists of the amino acid sequence SSTQAQ (SEQ ID NO:15). In other embodiments, the peptide comprises or consists of the amino acid sequence STQAQQ (SEQ ID NO:16). In other embodiments, the peptide comprises or consists of the amino acid sequence TQAQQS (SEQ ID NO:17). In other embodiments, the peptide comprises or consists of the amino acid sequence QAQQSY (SEQ ID NO:18). In other embodiments, the peptide comprises or consists of the amino acid sequence QAQQSW (SEQ ID NO:19). In other embodiments, the peptide comprises or consists of the amino acid sequence QAQQSF (SEQ ID NO:20).

In some embodiments, the peptide comprises or consists of the amino acid sequence LSSTQAQ (SEQ ID NO:21). In other embodiments, the peptide comprises or consists of the amino acid sequence SSTQAQQ (SEQ ID NO:22). In other embodiments, the peptide comprises or consists of the amino acid sequence STQAQQS (SEQ ID NO:23). In other embodiments, the peptide comprises or consists of the amino acid sequence TQAQQSY (SEQ ID NO:24). In other embodiments, the peptide comprises or consists of the amino acid sequence TQAQQSW (SEQ ID NO:25). In other embodiments, the peptide comprises or consists of the amino acid sequence TQAQQSF (SEQ ID NO:26).

In some embodiments, the peptide comprises or consists of the amino acid sequence LSSTQAQQ (SEQ ID NO:27). In other embodiments, the peptide comprises or consists of the amino acid sequence SSTQAQQS (SEQ ID NO:28). In other embodiments, the peptide comprises or consists of the amino acid sequence STQAQQSY (SEQ ID NO:29). In other embodiments, the peptide comprises or consists of the amino acid sequence STQAQQSW (SEQ ID NO:30). In other embodiments, the peptide comprises or consists of the amino acid sequence STQAQQSF (SEQ ID NO:31).

In some embodiments, the peptide comprises or consists of the amino acid sequence SSTQAQQSW (SEQ ID NO:32). In other embodiments, the peptide comprises or consists of the amino acid sequence SSTQAQQSF (SEQ ID NO:33). In other embodiments, the peptide comprises the amino acid sequence LSSTQAQQS (SEQ ID NO:2). In other embodiments, the peptide comprises the amino acid sequence SSTQAQQSY (SEQ ID NO:3).

In some embodiments, a peptide of the disclosure has an amino acid having an aromatic side chain (e.g., phenylalanine, tryptophan, or tyrosine) at the and/or C-terminus of the peptide.

In some embodiments, the peptide has a C-terminal tryptophan.

The peptides can be entirely L-amino acids, entirely D-amino acids, or a mixture of L-amino acids and D-amino acids, with peptides that are entirely L-amino acids being preferred. Peptides containing two or more asymmetric carbon atoms can be any form of mixtures of enantiomers or diastereomers in any ratio.

The peptides can be in the form of salts, preferably comprising counterions that are pharmaceutically acceptable (e.g., chloride, sulfate, citrate, phosphate, acetate, sodium, potassium, calcium, magnesium). Peptide salts can be acid addition salts or a base addition salts. Exemplary acids that can be used to make an acid addition salt include hydrochloric acid, sulfuric acid, nitric acid, phosphoric acid, hydrobromic acid, perchloric acid, citric acid, succinic acid, maleic acid, fumaric acid, malic acid, tartaric acid, p-toluenesulfonic acid, benzenesulfonic acid, methanesulfonic acid, and trifluoroacetic acid. Exemplary bases that can be used to make a base addition salt include sodium hydroxide, potassium hydroxide, bases of alkali metals, such as lithium hydroxide, calcium hydroxide, and bases of alkaline earth metal salts such as magnesium hydroxide. Additional acids and bases that can be used to make pharmaceutically acceptable salts are described in Stahl and Wermuth, eds., 2008, Handbook of Pharmaceutical Salts: Properties, Selection and Use, Verlag Helvetica Chimica Acta, Zurich, Switzerland.

The peptides be obtained, for example, by liquid phase peptide synthesis or solid phase peptide synthesis according to methods known in the art (e.g., as described in Benoiton, N., 2006, Chemistry of Peptide Synthesis, CRC Press, Boca Raton, FL; Howl, J., ed., 2005, Peptide Synthesis and Applications, Humana Press, Totowa, NJ; Chan and White, eds., 2000, Fmoc Solid Phase Synthesis: A Practical Approach, Oxford University Press, Oxford, UK). Custom peptide synthesis is also available commercially from numerous vendors (e.g., ABI Scientific (Sterling, VA); AnaSpec (Freemont CA); Pepscan, (Lelystad, Netherlands), Neo Scientific (Cambridge, MA); Sigma-Aldrich (St. Louis, MO)).

### 5.2. Peptide Conjugates

The disclosure provides peptide conjugates and salts thereof that comprise a peptide moiety and a conjugate moiety as recited in claim 4. Attachment of a conjugate moiety to a peptide can provide, for example, improved water solubility, improved stability, and reduced clearance as compared to the non-conjugated peptide (Hamley, 2014, Biomacromolecules 15:1543-1559). Thus, peptide conjugates can in some instances be more suitable as therapeutic agents compared to their unconjugated counterparts. Exemplary peptide moieties are described in Section 5.2.1 and exemplary conjugate moieties are described in Section 5.2.2. It should be understood that when an embodiment described herein refers to a "peptide conjugate," the embodiment encompasses the peptide conjugate per se as well as salts of the peptide conjugate even though the embodiment may not explicitly recite the expression "or salt thereof' or similar, unless required otherwise by context. Exemplary salts are described in Section 5.2.3.

### 5.2.1. Peptide Moieties

The peptide moiety of the conjugate of the invention is defined in claim 4.

The peptide moieties of the disclosure are 5 to 11 amino acids (i.e., 5, 6, 7, 8, 9, 10 or 11 amino acids) in length. In some embodiments, a peptide moiety of the disclosure is 5 amino acids in length. In other embodiments, a peptide moiety of the disclosure is 6 amino acids in length. In other embodiments, a peptide moiety of the disclosure is 7 amino acids in length. In other embodiments, a peptide moiety of the disclosure is 8 amino acids in length. In other embodiments, a peptide moiety of the disclosure is 9 amino acids in length. In other embodiments, a peptide moiety of the disclosure is 10 amino acids in length. In other embodiments, a peptide moiety of the disclosure is 11 amino acids in length.

In some embodiments, the peptide moiety comprises or consists of the amino acid sequence LSSTQ (SEQ ID NO:4). In other embodiments, the peptide moiety comprises or consists of the amino acid sequence SSTQA (SEQ ID NO:8). In other embodiments, the peptide moiety comprises or consists of the amino acid sequence STQAQ (SEQ ID NO:9). In other embodiments, the peptide moiety comprises or consists of the amino acid sequence TQAQQ (SEQ ID NO:10). In other embodiments, the peptide moiety comprises or consists of the amino acid sequence QAQQS (SEQ ID NO:11). In other embodiments, the peptide moiety comprises or consists of the amino acid sequence AQQSY (SEQ ID NO:5). In other embodiments, the peptide moiety comprises or consists of the amino acid sequence AQQSW (SEQ ID NO:12). In other embodiments, the peptide moiety comprises or consists of the amino acid sequence AQQSF (SEQ ID NO:13).

In some embodiments, the peptide moiety comprises or consists of the amino acid sequence LSSTQA (SEQ ID NO:14). In other embodiments, the peptide moiety comprises or consists of the amino acid sequence SSTQAQ (SEQ ID NO:15). In other embodiments, the peptide moiety comprises or consists of the amino acid sequence STQAQQ (SEQ ID NO:16). In other embodiments, the peptide moiety comprises or consists of the amino acid sequence TQAQQS (SEQ ID NO:17). In other embodiments, the peptide moiety comprises or consists of the amino acid sequence QAQQSY (SEQ ID NO:18). In other embodiments, the peptide moiety comprises or consists of the amino acid sequence QAQQSW (SEQ ID NO:19). In other embodiments, the peptide moiety comprises or consists of the amino acid sequence QAQQSF (SEQ ID NO:20).

In some embodiments the peptide moiety comprises or consists of the amino acid sequence LSSTQAQ (SEQ ID NO:21). In other embodiments, the peptide moiety comprises or consists of the amino acid sequence SSTQAQQ (SEQ ID NO:22). In other embodiments, the peptide moiety comprises or consists of the amino acid sequence STQAQQS (SEQ ID NO:23). In other embodiments, the peptide moiety comprises or consists of the amino acid sequence TQAQQSY (SEQ ID NO:24). In other embodiments, the peptide moiety comprises or consists of the amino acid sequence TQAQQSW (SEQ ID NO:25). In other embodiments, the peptide moiety comprises or consists of the amino acid sequence TQAQQSF (SEQ ID NO:26).

In some embodiments, the peptide moiety comprises or consists of the amino acid sequence LSSTQAQQ (SEQ ID NO:27). In other embodiments, the peptide moiety comprises or consists of the amino acid sequence SSTQAQQS (SEQ ID NO:28). In other embodiments, the peptide moiety comprises or consists of the amino acid sequence STQAQQSY (SEQ ID NO:29). In other embodiments, the peptide moiety comprises or consists of the amino acid sequence STQAQQSW (SEQ ID NO:30). In other embodiments, the peptide moiety comprises or consists of the amino acid sequence STQAQQSF (SEQ ID NO:31).

In some embodiments, the peptide moiety comprises or consists of the amino acid sequence LSSTQAQQS (SEQ ID NO:2). In other embodiments, the peptide moiety comprises or consists of the amino acid sequence SSTQAQQSY (SEQ ID NO:3). In other embodiments, the peptide moiety comprises or consists of the amino acid sequence SSTQAQQSW (SEQ ID NO:32). In other embodiments, the peptide moiety comprises or consists of the amino acid sequence SSTQAQQSF (SEQ ID NO:33).

In some embodiments, a peptide moiety of the disclosure has an amino acid having an aromatic side chain (e.g., phenylalanine, tryptophan, or tyrosine) at the and/or C-terminus of the peptide. In some embodiments, the peptide moiety has a C-terminal tryptophan.

The peptide moieties can be entirely L-amino acids, entirely D-amino acids, or a mixture of L-amino acids and D-amino acids, with peptide moieties that are entirely L-amino acids being preferred. Peptide moieties containing two or more asymmetric carbon atoms can be any form of mixtures of enantiomers or diastereomers in any ratio.

Peptide moieties can be obtained, for example, by liquid phase peptide synthesis or solid phase peptide synthesis according to methods known in the art (e.g., as described in Benoiton, N., 2006, Chemistry of Peptide Synthesis, CRC Press, Boca Raton, FL; Howl, J., ed., 2005, Peptide Synthesis and Applications, Humana Press, Totowa, NJ; Chan and White, eds., 2000, Fmoc Solid Phase Synthesis: A Practical Approach, Oxford University Press, Oxford, UK). Custom peptide synthesis is also available commercially from numerous vendors (e.g., ABI Scientific (Sterling, VA); AnaSpec (Freemont CA); Pepscan, (Lelystad, Netherlands), Neo Scientific (Cambridge, MA); Sigma-Aldrich (St. Louis, MO)).

### 5.2.2. Conjugate Moieties

The peptide conjugates comprise one or more conjugate moieties (e.g., 1, 2, 3, 4, or 5 conjugate moieties) attached to the peptide moiety. The conjugate moiety or moieties can be attached to an N-terminal amino acid, a C-terminal amino acid, an amino acid that is neither an N-terminal amino acid or a C-terminal amino acid, or a combination thereof. For example, a peptide conjugate can comprise one conjugate moiety, preferably which is either attached to the N-terminal amino acid of the peptide moiety or attached to the C-terminal amino acid of the peptide moiety. As another example, a peptide conjugate can comprise two conjugate moieties, one of which is preferably attached to the N-terminal amino acid of the peptide moiety and the other of which is preferably attached to the C-terminal amino acid of the peptide moiety.

In embodiments in which the peptide conjugate comprises multiple conjugate moieties, each of the conjugate moieties can be the same, some of the conjugate moieties can be the same and others can be different, or all of the conjugate moieties can be different. For example, a peptide conjugate having two conjugate moieties can have two of the same conjugate moiety. Alternatively, a peptide conjugate having two conjugate moieties can have two different conjugate moieties. As another example, a peptide conjugate having three conjugate moieties can have three of the same conjugate moiety, three different conjugate moieties, or two of the same conjugate moiety and one different conjugate moiety.

A conjugate moiety can be attached to a peptide moiety, for example, at one of the peptide moiety's amino acid side chains, its backbone, its N-terminal amino group, or its C-terminal carboxylic acid group. For example, a conjugate moiety can be attached to an amino acid side chain to form a chemically modified amino acid, such as methionine sulfoxide, methionine sulfone, S-(carboxymethyl)-cysteine, S-(carboxymethyl)-cysteine sulfoxide and S-(carboxymethyl)-cysteine sulfone. Other side chain modifications include acylation of lysine ε-amino groups, N-alkylation of arginine, histidine, or lysine, and alkylation of glutamic or aspartic carboxylic acid groups. Conjugate moieties can be attached to the peptide backbone, for example to a nitrogen atom in the backbone (e.g., a methyl conjugate moiety can be introduced into a peptide conjugate's backbone by using an N-methyl amino acid to synthesize the peptide). Conjugate moieties can be attached to the N-terminal amino group of the peptide moiety to provide, for example, an N-terminus having an N-lower alkyl, N-di-lower alkyl, or N-acyl modifications. Conjugate moieties can be attached to the C-terminal carboxy group to provide, for example, a peptide conjugate having an amide, a lower alkyl amide, a dialkyl amide, or a lower alkyl ester at the C-terminus of the conjugate. A lower alkyl refers to a C₁-C₄ alkyl.

Exemplary conjugate moieties that can be used in the peptide conjugates include polymers, amine groups (e.g., amino (-NH₂), alkyl amino and dialkyl amino), acyls groups (e.g., formyl or acetyl), alkyl groups (e.g., C₁-C₄ alkyl), phosphate groups, lipids and sugars.

In some embodiments, at least one, more than one, or all of the conjugate moieties in the peptide conjugate comprise(s) a polymer. Exemplary polymers that can be used as conjugate moieties include polyethylene glycol, polyvinyl pyrrolidone, polylactic-co-glycolic acid, N-(2-hydroxypropyl) methacrylamide copolymer, polyglutamic acid, and polysaccharides. In some embodiments, at least one, more than one, or all of the conjugate moieties in the peptide conjugate comprise(s) a polyethylene glycol. In some embodiments, at least one, more than one, or all of the conjugate moieties in the peptide conjugate comprise(s) polyvinyl pyrrolidone. In some embodiments, at least one, more than one, or all of the conjugate moieties in the peptide conjugate comprise(s) polylactic-co-glycolic acid. In some embodiments, at least one, more than one, or all of the conjugate moieties in the peptide conjugate comprise(s) N-(2-hydroxypropyl) methacrylamide copolymer. In some embodiments, at least one, more than one, or all of the conjugate moieties in the peptide conjugate comprise(s) polyglutamic acid. In some embodiments, at least one, more than one, or all of the conjugate moieties in the peptide conjugate comprise(s) comprises a polysaccharide.

In some embodiments, at least one, more than one, or all of the conjugate moieties in the peptide conjugate comprise(s) an amine group. Exemplary amine groups include amino (-NH₂), alkyl amino, and dialkyl amino groups. The alkyl groups can be, for example, a C₁-C₄ alkyl. In some embodiments, at least one, more than one, or all of the conjugate moieties in the peptide conjugate comprise(s) an amino group. In some embodiments, at least one, more than one, or all of the conjugate moieties in the peptide conjugate comprise(s) an alkyl amino group. In some embodiments, at least one, more than one, or all of the conjugate moieties in the peptide conjugate comprise(s) a dialkyl amino group.

In some embodiments, at least one, more than one, or all of the conjugate moieties in the peptide conjugate comprise(s) an acyl group. Exemplary acyl groups include formyl groups and acetyl groups. In some embodiments, at least one, more than one, or all of the conjugate moieties in the peptide conjugate comprise(s) a formyl group. In some embodiments, at least one, more than one, or all of the conjugate moieties in the peptide conjugate comprise(s) an acetyl group.

In some embodiments, at least one, more than one, or all of the conjugate moieties in the peptide conjugate comprise(s) an alkyl group. In exemplary embodiments, the alkyl group is a lower alkyl group, such as methyl or ethyl. In some embodiments, at least one, more than one, or all of the conjugate moieties in the peptide conjugate comprise(s) a methyl group. In some embodiments, at least one, more than one, or all of the conjugate moieties in the peptide conjugate comprise(s) an ethyl group.

In some embodiments, at least one, more than one, or all of the conjugate moieties in the peptide conjugate comprise(s) a phosphate group, for example attached to the side chain of a serine, threonine, or tyrosine.

In some embodiments, at least one, more than one, or all of the conjugate moieties in the peptide conjugate comprise(s) a lipid.

In some embodiments, at least one, more than one, or all of the conjugate moieties in the peptide conjugate comprise(s) a sugar.

In some embodiments, the peptide conjugate comprises one or more of the peptide modifications described in PCT international application no. PCT/JP2016/056453.

Processes for attaching conjugate moieties to peptide moieties are known in the art and can be used to obtain the peptide conjugates described herein (e.g., as described in Basle et al., 2010, Chemistry & Biology 17:213-227; Benoiton, N., 2006, Chemistry of Peptide Synthesis, CRC Press, Boca Raton, FL; Ernst and Leumann, eds., 1995, Modern Synthetic Methods, Verlag Helvetica Chimica Acta, Basel, Switzerland; Hamley, 2014, Biomacromolecules 15:1543-1559; Lundblad, R., 1995, Techniques in Protein Modification, CRC Press, Boca Raton, FL). Custom synthesis of peptide conjugates is also commercially available from numerous vendors (e.g., ABI Scientific (Sterling, VA); AnaSpec (Freemont CA); Pepscan, (Lelystad, Netherlands), Neo Scientific (Cambridge, MA); Sigma-Aldrich (St. Louis, MO), variously offering, for example, peptides having N-terminal conjugate moieties such as an acetyl group, a formyl group, a fatty acid, and alkyl amino groups, peptides having C-terminal conjugate moieties such as an amido group, alkyl amino groups, and alkyl groups, peptides conjugated to fatty acids, peptides conjugated to polyethylene glycol, and peptides having a phosphate conjugate moiety (e.g., comprising phosphoserine, phosphothreonine, or phosphotyrosine)).

### 5.2.3. Salts of Peptide Conjugates

The peptide conjugates can be in the form of salts, preferably comprising counterions that are pharmaceutically acceptable (e.g., chloride, sulfate, citrate, phosphate, acetate, sodium, potassium, calcium, magnesium). The peptide conjugate salts can be an acid addition salt or a base addition salt. Exemplary acids that can be used to make an acid addition salt include hydrochloric acid, sulfuric acid, nitric acid, phosphoric acid, hydrobromic acid, perchloric acid, citric acid, succinic acid, maleic acid, fumaric acid, malic acid, tartaric acid, p-toluenesulfonic acid, benzenesulfonic acid, methanesulfonic acid, and trifluoroacetic acid. Exemplary bases that can be used to make a base addition salt include sodium hydroxide, potassium hydroxide, bases of alkali metals, such as lithium hydroxide, calcium hydroxide, and bases of alkaline earth metal salts such as magnesium hydroxide. Additional acids and bases that can be used to make pharmaceutically acceptable salts are described in Stahl and Wermuth, eds., 2008, Handbook of Pharmaceutical Salts: Properties, Selection and Use, Verlag Helvetica Chimica Acta, Zurich, Switzerland.

### 5.3. Pharmaceutical Compositions

The peptides, salts thereof, peptide conjugates, and salts thereof described in Sections 5.1 and 5.2 can be formulated into pharmaceutical compositions comprising one or more pharmaceutically acceptable carriers, diluents, and/or excipients.

The pharmaceutical compositions can be formulated for topical, enteral (e.g., oral or rectal) or parenteral (e.g., intramuscular or intravenous) administration. Preferably, the pharmaceutical compositions are formulated for oral administration. The pharmaceutical compositions can be formulated as any suitable dosage form, such as a tablet, a capsule, granules, a powder, a syrup, a suspension, a suppository, an ointment, a cream, a gel, a patch, an inhalation, a solution for injection and the like according to techniques known in the art (e.g., as described in Allen et al., eds., 2012, Remington: The Science and Practice of Pharmacy, 22nd Edition, Pharmaceutical Press, London, UK).

Liquid pharmaceutical compositions can comprise a suitable solvent such as water, saline, a glucose solution, or ethanol. Liquid pharmaceutical composition can be formulated, for example, by dissolving or suspending an amount of a compound of the disclosure in the solvent. Buffering agents, preservatives, flavoring agents and colorants can each optionally be included in liquid formulations. Solid pharmaceutical compositions can include binders, lubricants, disintegrating agents, coloring agents, flavoring agents, flow-inducing agents, and melting agents. For example, solid formulations can contain an inert carrier such as lactose, gelatin, agar, starch, sucrose, glucose, methyl cellulose, dicalcium phosphate, calcium sulfate, mannitol, sorbitol, crystalline cellulose and the like. Binders include starch, gelatin, sugars such as glucose, lactose and trehalose, corn starch, calcium lactate, natural and synthetic gums such as acacia, tragacanth, or sodium alginate, povidone, carboxymethylcellulose, hydroxypropyl cellulose, polyethylene glycol, waxes, and the like. Lubricants include, for example, sodium oleate, sodium stearate, magnesium stearate, stearic acid, sodium stearyl fumarate, anhydrous silicic acid, talc and the like. Disintegrators include, for example, starch, methyl cellulose, agar, bentonite, xanthan gum, croscarmellose sodium, sodium starch glycolate and the like. Solid pharmaceutical compositions can be enteric coated, for example, with methyl methacrylate polymer, ethyl cellulose, or carnauba wax. Additional agents that can be included in the pharmaceutical compositions are described in PCT international application no. PCT/JP2016/056453.

The compounds of the disclosure can be included in pharmaceutical compositions in any suitable amount. Typically, a compound of the disclosure constitutes 0.1 wt% to less than 100 wt% of the pharmaceutical composition (e.g., 1 wt% to 99 wt%, 1 wt% to 90 wt%, 5 wt% to 80 wt%, 10 wt% to 75 wt%, or 15 wt% to 50 wt% of the pharmaceutical composition, or any wt% range bound by any two of the foregoing values).

### 5.4. Food Products

The peptides, salts thereof, peptide conjugates, and salts thereof described in Sections 5.1 and 5.2 can be formulated into food products such as a dietary supplement or a functional food.

The dietary supplement can be, for example, in the form of a tablet, a capsule, a softgel, a gelcap, a liquid, or a powder. Vitamins, minerals, herbs or other botanicals, amino acids, proteins, fiber, fatty acids, or a combination thereof can be included in the supplement.

Functional foods are foods in which a compound of the disclosure has been added or incorporated. For example, the functional food can be a drink such as coffee, cocoa, juice, a soft drink, a mineral beverage, a tea beverage, green tea, black tea, oolong tea, a milk drink, a lactic acid bacteria drink, a yogurt drink, a carbonated beverage, a nonalcoholic beverage, or an alcoholic beverage. The functional food can be a confectionery (e.g., a hard candy, a gum, a gummy, a jelly, a pudding, a mousse, a cake, a candy, a cookie, a cracker, a biscuit, a chocolate, a frozen dessert such as ice cream, ice candy, sherbet, shaved ice and the like), a dressing, a seasoning, a soy processed food (e.g., tofu, miso, soy sauce, bean curd, soybean flour, natto, etc.), a meat processed food (e.g., hamburger, meatloaf, meatballs, etc.), a fish meat processed food (e.g., boiled fish paste, such as fish sausage), a jelly-like food (e.g., jelly, agar, a jelly-like beverage, etc.), and the like.

### 5.5. Methods of Treatment

The peptides, salts thereof, peptide conjugates, and salts thereof described in Sections 5.1 and 5.2, the pharmaceutical compositions described in Section 5.3, and the food products described in Section 5.4, can be used to treat or prevent a mood disorder. Mood disorders that can be treated or prevented include depression, bipolar disorder, and adjustment disorder.

The disclosure provides a method for treating a subject suffering from a mood disorder comprising administering to the subject a compound of the disclosure or a pharmaceutical composition comprising a compound of the disclosure in an amount effective to treat the subject. The disclosure also provides a method for treating or preventing a mood disorder comprising administering an amount of a food product comprising a compound of the disclosure to a subject prone to or suffering from a mood disorder. In some embodiments, the subject is prone to suffer from a mood disorder. In other embodiments, the subject is suffering from a mood disorder.

In some embodiments of the methods described in the preceding paragraph, the mood disorder comprises depression. In some embodiments of the methods described in the preceding paragraph, the mood disorder comprises bipolar disorder. In some embodiments of the methods described in the preceding paragraph, the mood disorder comprises adjustment disorder.

The peptides, salts thereof, peptide conjugates, and salts thereof described in Sections 5.1 and 5.2, the pharmaceutical compositions described in Section 5.3, and the food products described in Section 5.4, can be used to treat or prevent an anxiety disorder.

The disclosure provides a method for treating a subject suffering from an anxiety disorder comprising administering to the subject a compound of the disclosure or a pharmaceutical composition comprising a compound of the disclosure in an amount effective to treat the subject. The disclosure also provides a method for treating or preventing an anxiety disorder comprising administering an amount of a food product comprising a compound of the disclosure to a subject prone to or suffering from the anxiety disorder. In some embodiments, the subject is prone to suffer from an anxiety disorder. In other embodiments, the subject is suffering from an anxiety disorder.

The peptides, salts thereof, peptide conjugates, and salts thereof described in Sections 5.1 and 5.2, the pharmaceutical compositions described in Section 5.3, and the food products described in Section 5.4, can be used to treat or prevent disorder of diminished motivation. Disorders of diminished motivation that can be treated or prevented include apathy, abulia, and akinetic mutism.

The disclosure provides a method for treating a subject suffering from a disorder of diminished motivation comprising administering to the subject a compound of the disclosure in an amount effective to treat the subject. The disclosure also provides a method for treating or preventing a disorder of diminished motivation comprising administering an amount of a food product comprising a compound of the disclosure to a subject prone to or suffering from a disorder of diminished motivation. In some embodiments, the subject is prone to suffer from a disorder of diminished motivation. In other embodiments, the subject is suffering from a disorder of diminished motivation.

In some embodiments of the methods described in the preceding paragraph, the disorder of diminished motivation comprises apathy. In other embodiments of the methods described in the preceding paragraph, the disorder of diminished motivation comprises abulia. In other embodiments of the methods described in the preceding paragraph, the disorder of diminished motivation comprises akinetic mutism.

The subjects of the methods described herein are preferably mammals, e.g., humans or domestic pets (e.g., cat, dog). Subjects can be of any age, but are preferably adults (e.g., a human subject who is 18 years old or more, 25 years old or more, 35 years old or more, 45 years old or more, 55 years old or more, etc.). In some embodiments, the subject is elderly (e.g., a human subject who is 65 years old or more, 70 years old or more, 75 years old or more, or 80 years old or more).

The compounds of the disclosure and pharmaceutical compositions containing them can be administered orally, topically, rectally, or parenterally, with oral administration being preferred. The method of administration can vary depending on the condition and age of the subject.

Appropriate daily dosages of the compounds of the disclosure can range from 0.005 mg/kg to 500 mg/kg of body weight per day (e.g., 0.005 mg/kg to 100 mg/kg, 0.005 mg/kg to 30 mg/kg, 0.005 mg/kg to 1 mg/kg, 0.01 mg/kg to 30 mg/kg, 0.01 mg/kg to 3 mg/kg, 0.01 mg/kg to 1 mg/kg, 0.02 mg/kg to 5 mg/kg, 0.02 mg/kg to 2 mg/kg, 0.02 mg/kg to 1 mg/kg). Alternatively, the compounds can be administered at a fixed dose ranging from 0.1 mg to 50 g per day (e.g., 0.1 mg to 10 g, 0.1 mg to 3 g, 0.1 mg to 100 mg, 0.1 mg to 1 mg, 0.3 mg to 3 g, or 0.3 mg to 100 mg). For administration of a pharmaceutical composition containing a compound of the disclosure, an amount of the pharmaceutical composition can be administered that contains an amount of the compound that is within one of the foregoing ranges. Likewise, for administration of one or more food products containing the compound, an amount of one or more food products can be administered that contain an amount of the compound that is within one of the foregoing ranges.

### 6. EXAMPLES

### 6.1. Methods

### 6.1.1. Tail suspension test

The tail suspension test is an experimental method used to screen potential antidepressant drugs (Can et al., 2012, J Vis Exp., 59:e3769). In the test as implemented in the Examples, a test substance is orally administered to mice (ddY mice, males, 24 - 30 g) and 30 minutes later the mice are suspended by their tails for six minutes. The amount of time that a mouse is immobile (i.e. is not displaying escape behavior) during the six minutes is measured to provide an immobility time. The administration of antidepressant drugs such as imipramine reduces immobility time. Therefore, if a reduction of immobility time is observed when testing a test substance, it can be concluded that the test substance has anti-depressant properties. Immobility is considered a despair state, and, therefore, a reduction of immobility time also indicates an increase in motivation (i.e., that the test substance has motivation-increasing properties).

### 6.1.2. Forced swimming test

The forced swimming test, like the tail suspension test, is an experimental method used to screen potential antidepressant drugs (Can et al., 2012 J Vis Exp., 59:e3638). In the test as implemented in the Examples, a test substance is orally administered to mice (ddY mice, males, 24 ~ 30 g) and 30 minutes later the mice are placed in an impossible to escape water bath for eight minutes. The amount of time that a mouse is immobile during the eight minutes is measured to provide an immobility time.

Like the tail suspension test, the administration of antidepressants, such as imipramine, reduces the immobility time in the forced swimming test. Therefore, if a reduction of immobility time is observed when testing a test substance, it can be it can be concluded that the test substance has anti-depressant properties. A reduction of immobility time also indicates an increase in motivation (i.e., that the test substance has motivation-increasing properties).

### 6.1.3. Elevated plus maze test

The elevated plus maze test is a widely used test for measuring anxiety-like behavior in mice and is based on the natural aversion of mice for open and elevated areas (Komada et al., 2008 J. Vis Exp., 22:e1088). The test can be used to assess the antianxiety effects of test substances. In the test as implemented in the Examples, an apparatus consisting of two open arms (25 cm × 5 cm) and two closed arms (25 cm × 5 cm × 15 cm) is positioned 50 cm above the floor. Despite the high position, a mouse can walk safely around the closed arms due to the high (15 cm) wall. On the other hand, a mouse walking the open arms feels a sense of anxiety because there is no wall protecting the mouse from falling. Thus, the longer the mouse is in the open arms, or the greater the frequency of entrance into the open arms, as compared to control, is an indication of anxiolytic activity of the test substance.

Mice (ddY mice, males, 24 - 30 g) are administered a test substances 30 minutes prior to testing. At the beginning of the test, a mouse is placed on the apparatus facing one of the open arms. During the 5-minute test time, the cumulative time spent in the open arms (time in open arms), the number of times a visit to the open arms is made (visit to open arms), and the total number of times a visit to one of the arms is made (total visits) is recorded.

### 6.1.4. Open field test

The open field test is used to measure anxiety-like behavior in mice (Bailey and Crawley, 2009, Anxiety-Related Behaviors in Mice, Ch. 5 in Methods of Behavior Analysis in Neurscience, 2nd edition, CRC Press, Boca Raton FL). Mice prefer the peripheral part of an enclosed area (such as an enclosed circle), and searching behavior to the central part is typically very small. In the test as implemented in the Examples, mice are administered a test substance 30 minutes prior to testing. At the start of the test, mice are placed in a circular chamber and their movement is observed for five minutes. The amount of time spent in the center circle of the chamber (12 cm), the number of visits to the center circle of the chamber, and the total distance traveled is measured. In mice administered the test substance, a higher proportion of time spent in the center circle as compared to control indicates anxiolytic activity of the test substance.

### 6.2. Example 1: Tail suspension test with peptide LSSTQAQQSY (SEQ ID NO:1)

The peptide LSSTQAQQSY (SEQ ID NO:1) was tested in a tail suspension test using mice orally administered 0.1mg/kg, 0.3mg/kg, 1 mg/kg or 3 mg/kg of the peptide (n = 8-10).

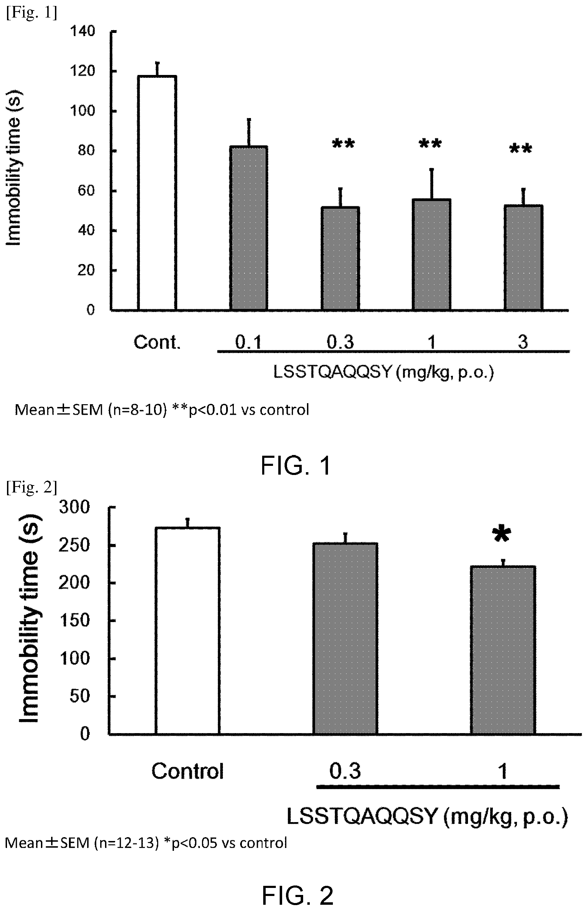

The results are shown in Figure 1. Immobility time was significantly reduced in the 0.3 mg/kg, 1 mg/kg, and 3 mg/kg groups as compared to saline control.

### 6.3. Example 2: Forced swimming test with peptide LSSTQAQQSY (SEQ ID NO:1)

The peptide LSSTQAQQSY (SEQ ID NO:1) was tested in a forced swimming test using mice orally administered 0.3 mg/kg or 1 mg/kg of the peptide (n = 12-13).

The results are shown in Figure 2. Immobility time was significantly reduced in 1mg/kg group as compared to saline control.

### 6.4. Example 3: Tail suspension test with peptides LSSTQAQQSY (SEQ ID NO:1), LSSTQAQQS (SEQ ID NO:2), SSTQAQQSY (SEQ ID NO:3), LSSTQ (SEQ ID NO:4), and AQQSY (SEQ ID NO:5)

Peptides LSSTQAQQSY (SEQ ID NO:1), LSSTQAQQS (SEQ ID NO:2) (corresponding to the first nine amino acids of SEQ ID NO:1), SSTQAQQSY (SEQ ID NO:3) (corresponding to the last nine amino acids of SEQ ID NO:1), LSSTQ (SEQ ID NO:4) (corresponding to the first five amino acids of SEQ ID NO:1), and AQQSY (SEQ ID NO:5) (corresponding to the last five amino acids of SEQ ID NO:1) were tested in a tail suspension test using mice orally administered 0.3 mg/kg of the peptides (n = 5-6 or 18-19).

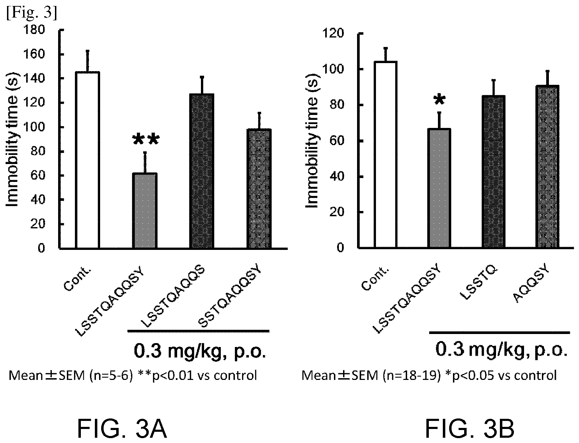

The results are shown in Figure 3. Immobility time was reduced in all of the test groups as compared to control, indicating that each peptide has antidepressant and motivation-increasing properties. The results were only statistically significant for the full length peptide LSSTQAQQSY (SEQ ID NO:1). The inventors had the insight that a more significant effect can be seen at higher doses of the shorter peptides (e.g., at 1 mg/kg, 3 mg/kg, or 10 mg/kg).

### 6.5. Example 4: Elevated plus maze test with peptide LSSTQAQQSY (SEQ ID NO:1)

The peptide LSSTQAQQSY (SEQ ID NO:1) was tested in an elevated plus maze test using mice orally administered 0.1 mg/kg, 0.3 mg/kg, or 1 mg/kg of the peptide (n = 18-19).

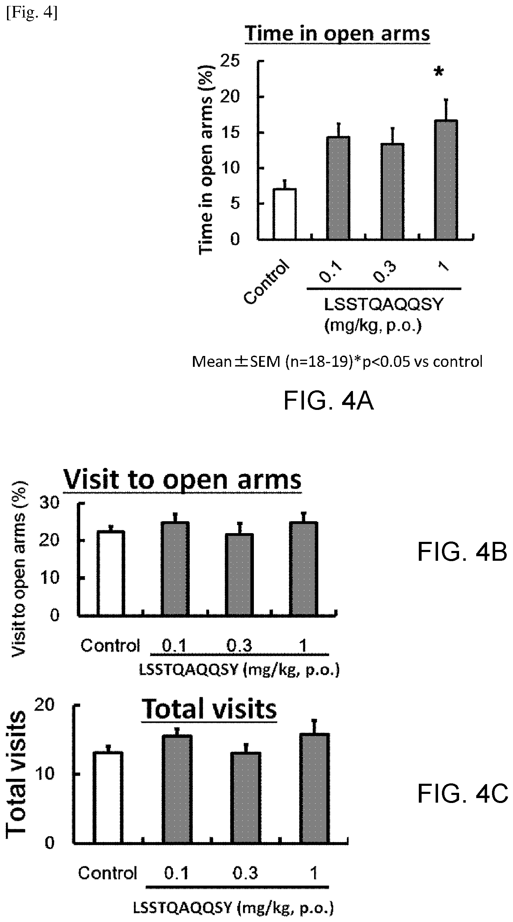

The results are shown in Figure 4. The percentage of time spent in the open arms was increased in all groups, and rose to the level of statistical significance for the 1 mg/kg group.

### 6.6. Example 5: Open field test with peptide LSSTQAQQSY (SEQ ID NO:1)

The peptide LSSTQAQQSY (SEQ ID NO:1) was tested in an open field test using mice orally administered 0.1 mg/kg, 0.3 mg/kg, or 1 mg/kg of the peptide (n = 4-5).

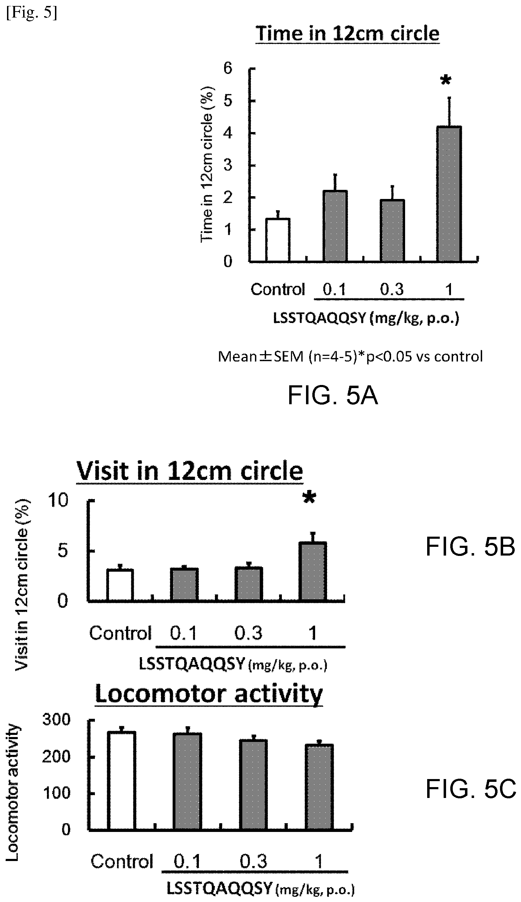

The results are shown in Figure 5. The amount of time that the mice spent in the center 12 cm circle was increased in all groups, and rose to the level of statistical significance in the 1 mg/kg group. A similar result was observed for the number of visits to the center 12 cm circle. On the other hand, the overall locomotor activity was not significantly different between the groups.

### 6.7. Example 6: Tail suspension test with the peptides LSSTQAQQSY (SEQ ID NO:1), LSSTQAQQSW (SEQ ID NO:6), and LSSTQAQQSF (SEQ ID NO:7), and the C-terminal amidated peptide conjugate LSSTQAQQSY-NH₂ (SEQ ID NO:36)

Peptides LSSTQAQQSY (SEQ ID NO:1), LSSTQAQQSW (SEQ ID NO: 6) and LSSTQAQQSF (SEQ ID NO: 7), and C-terminal amidated peptide conjugate LSSTQAQQSY-NH₂ (SEQ ID NO:36) were tested in a tail suspension test using mice orally administered 0.3 mg/kg of the peptides (n = 11-12).

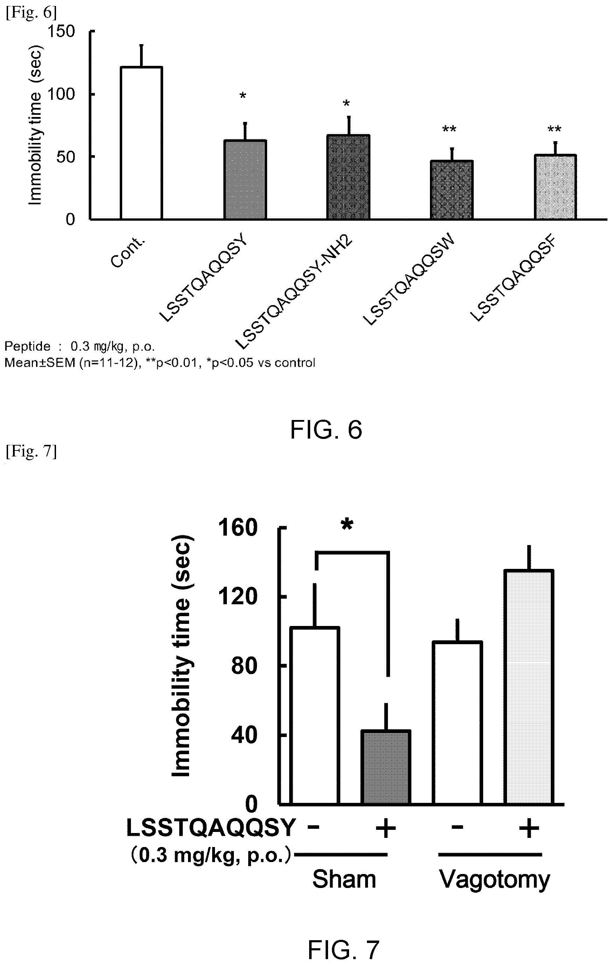

The results are shown in Figure 6. All of the peptides and the peptide conjugate significantly decreased immobility time, indicating that modifying the C-terminal end of the peptide LSSTQAQQSY (SEQ ID NO:1) by substituting the aromatic side chain (Y to W or F) or by adding an amino group maintains the anti-depressant and motivation-increasing activity of the peptide.

### 6.8. Example 7: Tail suspension test with five, six, seven, eight, and nine amino acid peptides

Five, six, seven, eight, and nine amino acid peptides SSTQA (SEQ ID NO:8), STQAQ (SEQ ID NO:9), TQAQQ (SEQ ID NO:10), QAQQS (SEQ ID NO:11), AQQSW (SEQ ID NO:12), or AQQSF (SEQ ID NO:13), LSSTQA (SEQ ID NO:14), SSTQAQ (SEQ ID NO:15), STQAQQ (SEQ ID NO:16), TQAQQS (SEQ ID NO:17), QAQQSY (SEQ ID NO:18), QAQQSW (SEQ ID NO:19), and QAQQSF (SEQ ID NO:20), LSSTQAQ (SEQ ID NO:21), SSTQAQQ (SEQ ID NO:22), STQAQQS (SEQ ID NO:23), TQAQQSY (SEQ ID NO:24), TQAQQSW (SEQ ID NO:25), TQAQQSF (SEQ ID NO:26), LSSTQAQQ (SEQ ID NO:27), SSTQAQQS (SEQ ID NO:28), STQAQQSY (SEQ ID NO:29), STQAQQSW (SEQ ID NO:30), STQAQQSF (SEQ ID NO:31), SSTQAQQSW (SEQ ID NO:32) and SSTQAQQSF (SEQ ID NO:33) are tested in a tail suspension test using mice orally administered 1 mg/kg, 3 mg/kg, or 10 mg/kg of one of the peptides.

A decrease in immobility time is observed for the mice administered a five, six, seven, eight, or nine amino acid peptide. An increased response is observed with increasing peptide dose.

### 6.9. Example 8: Forced swimming test with five, six, seven, eight, and nine amino acid peptides

Five, six, seven, eight, and nine amino acid peptides SSTQA (SEQ ID NO:8), STQAQ (SEQ ID NO:9), TQAQQ (SEQ ID NO:10), QAQQS (SEQ ID NO:11), AQQSW (SEQ ID NO:12), or AQQSF (SEQ ID NO:13), LSSTQA (SEQ ID NO:14), SSTQAQ (SEQ ID NO:15), STQAQQ (SEQ ID NO:16), TQAQQS (SEQ ID NO:17), QAQQSY (SEQ ID NO:18), QAQQSW (SEQ ID NO:19), and QAQQSF (SEQ ID NO:20), LSSTQAQ (SEQ ID NO:21), SSTQAQQ (SEQ ID NO:22), STQAQQS (SEQ ID NO:23), TQAQQSY (SEQ ID NO:24), TQAQQSW (SEQ ID NO:25), TQAQQSF (SEQ ID NO:26), LSSTQAQQ (SEQ ID NO:27), SSTQAQQS (SEQ ID NO:28), STQAQQSY (SEQ ID NO:29), STQAQQSW (SEQ ID NO:30), STQAQQSF (SEQ ID NO:31), SSTQAQQSW (SEQ ID NO:32) and SSTQAQQSF (SEQ ID NO:33) are tested in a forced swimming test using mice orally administered 1 mg/kg, 3 mg/kg, or 10 mg/kg of one of the peptides.

A decrease in immobility time is observed for the mice administered a five, six, seven, eight, or nine amino acid peptide. An increased response is observed with increasing peptide dose.

### 6.10. Example 9: Elevated plus maze test with five, six, seven, eight, and nine amino acid peptides

Five, six, seven, eight, and nine amino acid peptides SSTQA (SEQ ID NO:8), STQAQ (SEQ ID NO:9), TQAQQ (SEQ ID NO:10), QAQQS (SEQ ID NO:11), AQQSW (SEQ ID NO:12), or AQQSF (SEQ ID NO:13), LSSTQA (SEQ ID NO:14), SSTQAQ (SEQ ID NO:15), STQAQQ (SEQ ID NO:16), TQAQQS (SEQ ID NO:17), QAQQSY (SEQ ID NO:18), QAQQSW (SEQ ID NO:19), and QAQQSF (SEQ ID NO:20), LSSTQAQ (SEQ ID NO:21), SSTQAQQ (SEQ ID NO:22), STQAQQS (SEQ ID NO:23), TQAQQSY (SEQ ID NO:24), TQAQQSW (SEQ ID NO:25), TQAQQSF (SEQ ID NO:26), LSSTQAQQ (SEQ ID NO:27), SSTQAQQS (SEQ ID NO:28), STQAQQSY (SEQ ID NO:29), STQAQQSW (SEQ ID NO:30), STQAQQSF (SEQ ID NO:31), SSTQAQQSW (SEQ ID NO:32) and SSTQAQQSF (SEQ ID NO:33) are tested in an elevated plus maze test using mice orally administered 1 mg/kg, 3 mg/kg, or 10 mg/kg of one of the peptides.

The percentage of time spent in the open arms is increased for the mice administered a five, six, seven, eight, or nine amino acid peptide. An increased response is observed with increasing peptide dose.

### 6.11. Example 10: Open field test with five, six, seven, eight, and nine amino acid peptides

Five, six, seven, eight, and nine amino acid peptides SSTQA (SEQ ID NO:8), STQAQ (SEQ ID NO:9), TQAQQ (SEQ ID NO:10), QAQQS (SEQ ID NO:11), AQQSW (SEQ ID NO:12), or AQQSF (SEQ ID NO:13), LSSTQA (SEQ ID NO:14), SSTQAQ (SEQ ID NO:15), STQAQQ (SEQ ID NO:16), TQAQQS (SEQ ID NO:17), QAQQSY (SEQ ID NO:18), QAQQSW (SEQ ID NO:19), and QAQQSF (SEQ ID NO:20), LSSTQAQ (SEQ ID NO:21), SSTQAQQ (SEQ ID NO:22), STQAQQS (SEQ ID NO:23), TQAQQSY (SEQ ID NO:24), TQAQQSW (SEQ ID NO:25), TQAQQSF (SEQ ID NO:26), LSSTQAQQ (SEQ ID NO:27), SSTQAQQS (SEQ ID NO:28), STQAQQSY (SEQ ID NO:29), STQAQQSW (SEQ ID NO:30), STQAQQSF (SEQ ID NO:31), SSTQAQQSW (SEQ ID NO:32) and SSTQAQQSF (SEQ ID NO:33) are tested in an open field test using mice orally administered 1 mg/kg, 3 mg/kg, or 10 mg/ kg of one of the peptides.

The amount of time that the mice spend in the center 12 cm circle is increased for the mice administered a five, six, seven, eight, or nine amino acid peptide. An increased response is observed with increasing peptide dose.

### 6.12. Example 11: Examination of mechanism of action

The peptide LSSTQAQQSY (SEQ ID NO:1) was orally administered at 0.3mg/kg to vagotomized mice and sham operated mice one week after surgery to investigate the mechanism of action of the peptide. The mice were then subjected to a tail suspension test.

The results are shown in FIG. 7. The vagotomy abolished anti-depressant-like effect of the orally administered peptide. These results suggest that the peptide acts on the gut, and its signal is transferred to the central nervous system via the vagus nerve. The inventors believe that the compounds of the disclosure are likely to share the same mechanism of action as the peptide LSSTQAQQSY (SEQ ID NO:1).

### 6.13. Example 12: Investigation of mediators involved in the anti-depressant effects of the peptide LSSTQAQQSY (SEQ ID NO:1)

The peptide LSSTQAQQSY (SEQ ID NO:1) was orally administered to mice at 0.3mg/kg, alone and in combination with orally administered antagonists of serotonin 5-HT_{1A}, dopamine D₁, or GABA_{A} receptors and subjected to tail suspension test to investigate the mediators involved in the anti-depressant effects of the peptide. WAY100135, an antagonist of the 5-HT1_{A} receptor was dosed at 10 mg/kg. SCH23390, an antagonist of the dopamine D₁ receptor was dosed at 30µg/kg. Bicuculline, an antagonist of the GABA_{A} receptor, was dosed at 30 mg/kg.

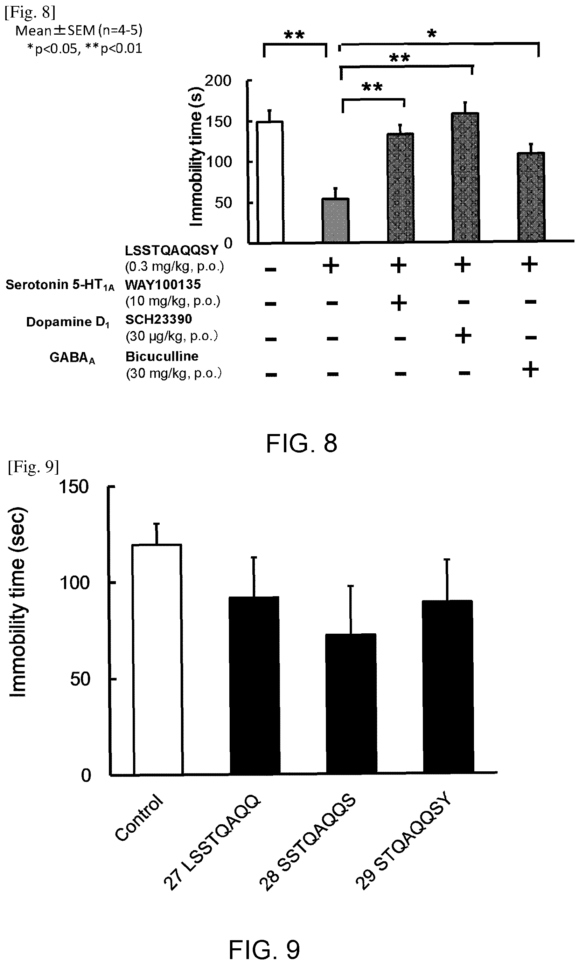

The results are shown in Figure 8. The peptide-induced antidepressant-like effect was blocked by the antagonists of serotonin 5-HT_{1A}, dopamine D₁, and GABA_{A} receptors. The peptide exhibited no affinity for these receptors, suggesting that it stimulates the release of these neurotransmitters. It was also determined using selective agonists and antagonists (data not shown) that the order of the receptor activation is 5-HT_{1A}, D₁, and GABA_{A}. Taken together, the peptide appears to exhibit an antidepressant-like effect after oral administration via activation of the brain-gut axis, and of 5-HT_{1A}, D₁, and GABA_{A} systems. The inventors believe that the compounds of the disclosure are likely to share the same mechanism of action as the peptide LSSTQAQQSY (SEQ ID NO:1).

### 6.14. Example 13: Tail suspension test with peptides LSSTQAQQ (SEQ ID NO:27), SSTQAQQS (SEQ ID NO:28), and STQAQQSY (SEQ ID NO:29)

Peptides LSSTQAQQ (SEQ ID NO:27), SSTQAQQS (SEQ ID NO:28), and STQAQQSY (SEQ ID NO:29) were tested in a tail suspension test using mice orally administered 0.3 mg/kg of the peptides (n = 5-6).

The results are shown in Figure 9. All of the peptides decreased immobility time compared to control.

### 6.15. Example 14: Tail suspension test with peptides LSSTQAQQSYW (SEQ ID NO:37), WLSSTQAQQSY (SEQ ID NO:38), WLSSTQAQQSYW (SEQ ID NO:39), WLSSTQ (SEQ ID NO:40), and AQQSYW (SEQ ID NO:41)

Peptides LSSTQAQQSYW (SEQ ID NO:37), WLSSTQAQQSY (SEQ ID NO:38), WLSSTQAQQSYW (SEQ ID NO:39), WLSSTQ (SEQ ID NO:40), and AQQSYW (SEQ ID NO:41) were tested in a tail suspension test using mice orally administered 0.3 mg/kg of the peptides (n = 3-5).

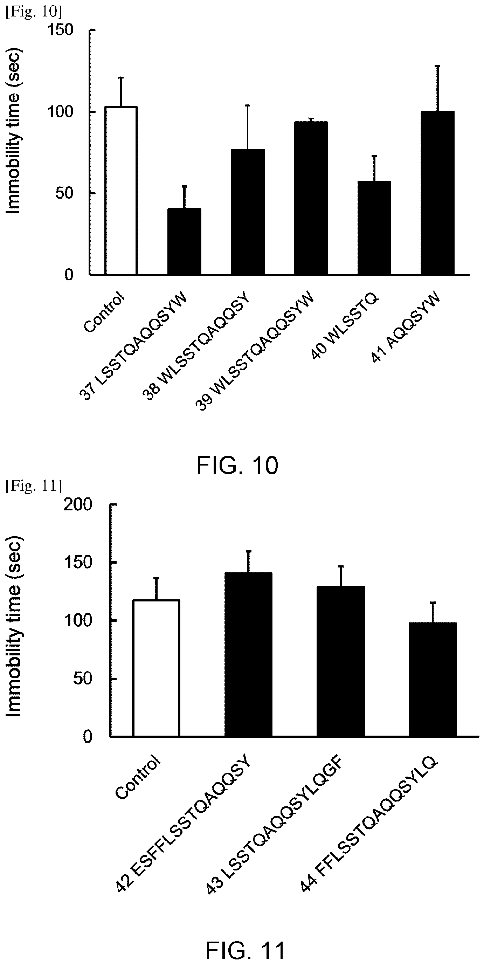

The results are shown in Figure 10. All of the peptides decreased immobility time compared to control.

### 6.16. Example 15: Tail suspension test with peptides ESFFLSSTQAQQSY (SEQ ID NO:42), LSSTQAQQSYLQGF (SEQ ID NO:43), and FFLSSTQAQQSYLQ (SEQ ID NO:44)

Peptides ESFFLSSTQAQQSY (SEQ ID NO:42), LSSTQAQQSYLQGF (SEQ ID NO:43), and FFLSSTQAQQSYLQ (SEQ ID NO:44) were tested in a tail suspension test using mice orally administered 0.3 mg/kg of the peptides (n = 6-7).

The results are shown in Figure 11. Peptide FFLSSTQAQQSYLQ (SEQ ID NO:44) decreased immobility time compared to control.

### 6.17. Example 16: Tail suspension test with peptides SSTQAQQS (SEQ ID NO:28), LSSTQAQQSYW (SEQ ID NO:37) and WLSSTQ (SEQ ID NO:40)

Peptides SSTQAQQS (SEQ ID NO:28), LSSTQAQQSYW (SEQ ID NO:37) and WLSSTQ (SEQ ID NO:40) were retested in a tail suspension test using mice orally administered 0.3 mg/kg of the peptides (n = 5-7).

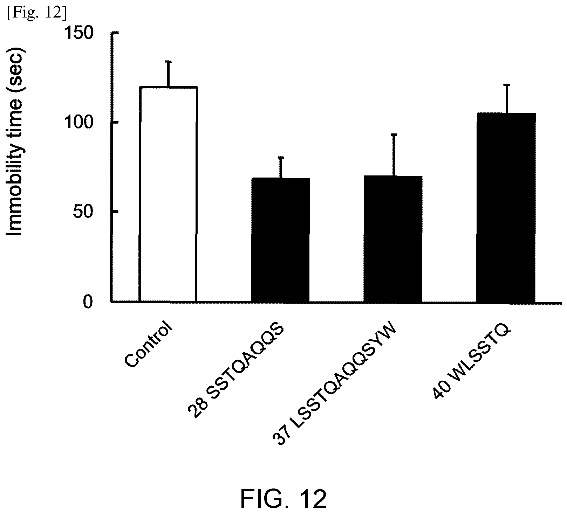

The results are shown in Figure 12. All of the peptides decreased immobility time compared to control.

### 6.18. Example 17: Analysis of tail suspension test data

The tail suspension test data from Examples 1, 3, 6, 13, 14, 15, and 16 were analyzed to identify correlations between sequence and activity. The tail suspension test data for the peptides were first normalized as a percentage of immobility time of the control in their respective study, such that lower percentages reflect greater activity. The results are shown in Table 1:

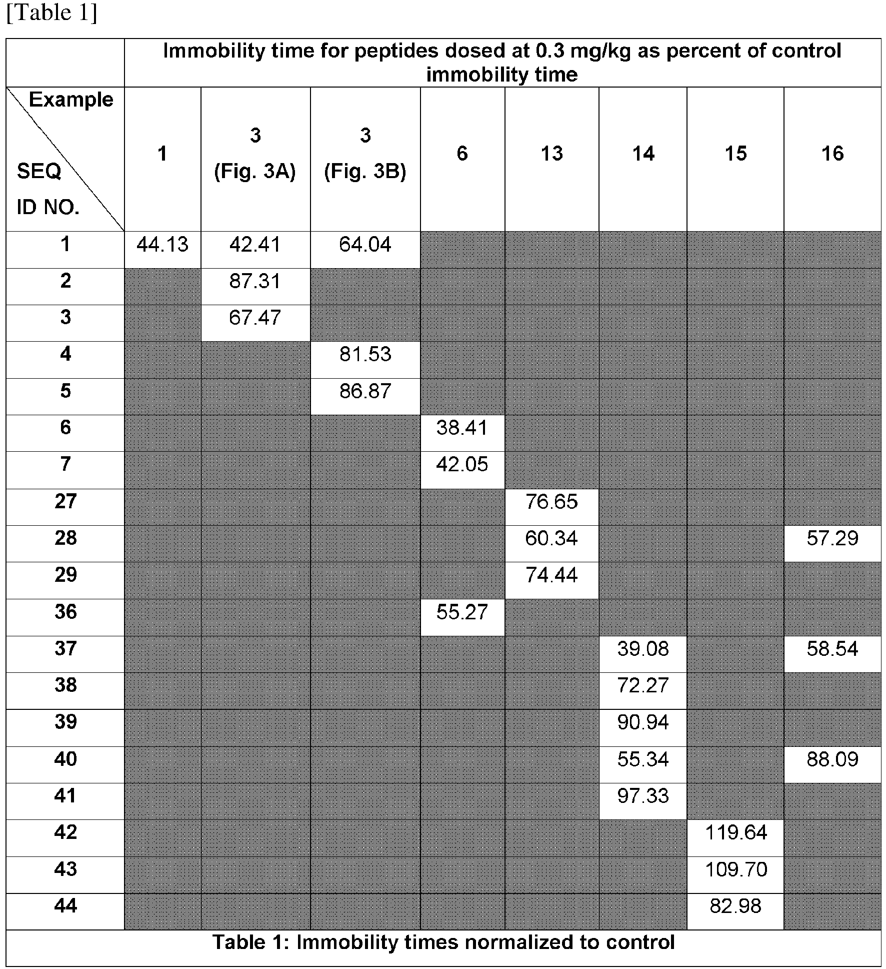

The normalized data for each of the peptides was then compared to the normalized data for peptide LSSTQAQQSY (SEQ ID NO:1) to rank the relative activities of the peptides. The percent reduction of control immobility was used to assign an activity rating of excellent, good, moderate, or low to each peptide. For peptides that were tested more than once (including SEQ ID NO:1, the reference peptide), the average reduction of control immobility was used in these calculations. The resulting activity ratings, together with amino acid alignments in which amino acid changes from SEQ ID NO:1 are underlined, are shown in Table 2.

**[Table 2]**

| **SEQ ID NO.** | **Sequence** | **% of SEQ ID NO:1 activity** | **Rank** | **Activity Rating** |
|---|---|---|---|---|
| 6 | LSSTQAQQS**W** | 122.71 | 1 | Excellent |
| 7 | LSSTQAQQS**F** | 115.46 | 2 | Excellent |
| 37 | LSSTQAQQSY**W** | 97.25 | 4 | Excellent |
| 1 | LSSTQAQQSY | 100 | 3 | Excellent |
| 36 | LSSTQAQQSY***** | 89.12 | 5 | Excellent |
| 28 | SSTQAQQS | 82.07 | 6 | Excellent |
| 3 | SSTQAQQSY | 64.81 | 7 | Excellent |
| 38 | **W**LSSTQAQQSY | 55.25 | 9 | Good |
| 29 | STQAQQSY | 50.93 | 10 | Good |
| 40 | **W**LSSTQ | 56.36 | 8 | Good |
| 27 | LSSTQAQQ | 46.52 | 11 | Good |
| 4 | LSSTQ | 36.80 | 12 | Good |
| 44 | **FF**LSSTQAQQSY**LQ** | 33.91 | 13 | Good |
| 5 | AQQSY | 26.16 | 14 | Moderate |
| 2 | LSSTQAQQS | 25.28 | 15 | Moderate |
| 39 | **W**LSSTQAQQSY**W** | 18.05 | 16 | Moderate |
| 41 | AQQSY**W** | 5.32 | 17 | Low |
| 43 | LSSTQAQQSY**LQGF** | -19.33 | 18 | Low |
| 42 | **ESFF**LSSTQAQQSY | -39.13 | 19 | Low |
| **Table 2: Peptide activity ratings** | | | | |

Without being bound by theory, the inventors believe that peptides comprising a core amino acid sequence of SSTQAQQS (SEQ ID NO:28) and having a total length of 8 to 12 amino acids (and more particularly 8 to 10 amino acids) are generally more active than peptides not comprising the SSTQAQQS (SEQ ID NO:28) core sequence, that an aromatic amino acid (Y, W or F) at one or both termini, particularly the C-terminus, increases activity, and that activity generally gradually decreases as peptide length is altered to fall outside these ranges.

### 8. SEQUENCE LISTING

**[Table 3]**

| **Sequence** | **length** | **SEQ ID NO** |
|---|---|---|
| LSSTQAQQSY | 10 | 1 |
| LSSTQAQQS | 9 | 2 |
| SSTQAQQSY | 9 | 3 |
| LSSTQ | 5 | 4 |
| AQQSY | 5 | 5 |
| LSSTQAQQSW | 10 | 6 |
| LSSTQAQQSF | 10 | 7 |
| SSTQA | 5 | 8 |
| STQAQ | 5 | 9 |
| TQAQQ | 5 | 10 |
| QAQQS | 5 | 11 |
| AQQSW | 5 | 12 |
| AQQSF | 5 | 13 |
| LSSTQA | 6 | 14 |
| SSTQAQ | 6 | 15 |
| STQAQQ | 6 | 16 |
| TQAQQS | 6 | 17 |
| QAQQSY | 6 | 18 |
| QAQQSW | 6 | 19 |
| QAQQSF | 6 | 20 |
| LSSTQAQ | 7 | 21 |
| SSTQAQQ | 7 | 22 |
| STQAQQS | 7 | 23 |
| TQAQQSY | 7 | 24 |

**[Table 4]**

| **Sequence** | **length** | **SEQ ID NO** |
|---|---|---|
| TQAQQSW | 7 | 25 |
| TQAQQSF | 7 | 26 |
| LSSTQAQQ | 8 | 27 |
| SSTQAQQS | 8 | 28 |
| STQAQQSY | 8 | 29 |
| STQAQQSW | 8 | 30 |
| STQAQQSF | 8 | 31 |
| SSTQAQQSW | 9 | 32 |
| SSTQAQQSF | 9 | 33 |
| LSSTQAQQSX₁, where X₁ is Y, W, or F | 10 | 34 |
| NKPGRFESFFLSSTQAQQSX₁ LQGFSKNILE, where X₁ is Y, W, or F | 30 | 35 |
| LSSTQAQQSY-NH₂ | 10 | 36 |
| LSSTQAQQSYW | 11 | 37 |
| WLSSTQAQQSY | 11 | 38 |
| WLSSTQAQQSYW | 12 | 39 |
| WLSSTQ | 6 | 40 |
| AQQSYW | 6 | 41 |
| ESFFLSSTQAQQSY | 14 | 42 |
| LSSTQAQQSYLQGF | 14 | 43 |
| FFLSSTQAQQSYLQ | 14 | 44 |

## Claims

1. A pharmaceutical composition comprising a compound that is a peptide or a salt thereof and one or more pharmaceutically acceptable carriers, diluents and/or excipients, wherein the amino acid sequence of the peptide:
a. consists of:
i. 5 to 10 consecutive amino acids from the amino acid sequence LSSTQAQQSX₁ (SEQ ID NO:34), where X₁ is Y, W, or F; and
ii. optionally, an additional aromatic amino acid at the C-terminus of said 5 to 10 consecutive amino acids; and
b. does not consist of the amino acid sequence LSSTQAQQSY (SEQ ID NO:1), LSSTQAQQS (SEQ ID NO:2), SSTQAQQSY (SEQ ID NO:3), LSSTQ (SEQ ID NO:4), AQQSY (SEQ ID NO:5), LSSTQAQQSW (SEQ ID NO:6), or LSSTQAQQSF (SEQ ID NO:7).

2. The pharmaceutical composition of claim 1, wherein the amino acid sequence of the peptide consists of:
a. 6 to 10 consecutive amino acids from the amino acid sequence LSSTQAQQSX₁ (SEQ ID NO:34) and, optionally, an additional aromatic amino acid at the C-terminus of said 6 to 10 consecutive amino acids;
b. 7 to 10 consecutive amino acids from the amino acid sequence LSSTQAQQSX₁ (SEQ ID NO:34) and, optionally, an additional aromatic amino acid at the C-terminus of said 7 to 10 consecutive amino acids; or
c. 8 to 10 consecutive amino acids from the amino acid sequence LSSTQAQQSX₁ (SEQ ID NO:34) and, optionally, an additional aromatic amino acid at the C-terminus of said 8 to 10 consecutive amino acids, optionally wherein said 8 to 10 consecutive amino acids consists of the amino acid sequence SSTQAQQS (SEQ ID NO:28).

3. The pharmaceutical composition of claim 1 or claim 2, wherein the peptide is 5 amino acids in length, 6 amino acids in length, 7 amino acids in length, 8 amino acids in length, 9 amino acids in length, 10 amino acids in length, or 11 amino acids in length, optionally wherein the peptide:
i. comprises or consists of the amino acid sequence SSTQA (SEQ ID NO:8);
ii. comprises or consists of the amino acid sequence STQAQ (SEQ ID NO:9);
iii. comprises or consists of the amino acid sequence TQAQQ (SEQ ID NO:10);
iv. comprises or consists of the amino acid sequence QAQQS (SEQ ID NO:11);
v. comprises or consists of the amino acid sequence AQQSW (SEQ ID NO:12);
vi. comprises or consists of the amino acid sequence or AQQSF (SEQ ID NO:13);
vii. consists of the amino acid sequence LSSTQ (SEQ ID NO:4) and an additional aromatic amino acid at the C-terminus;
viii. consists of the amino acid sequence AQQSY (SEQ ID NO:5) and an additional aromatic amino acid at the C-terminus;
ix. comprises or consists of the amino acid sequence LSSTQA (SEQ ID NO:14);
x. comprises or consists of the amino acid sequence SSTQAQ (SEQ ID NO:15);
xi. comprises or consists of the amino acid sequence STQAQQ (SEQ ID NO:16);
xii. comprises or consists of the amino acid sequence TQAQQS (SEQ ID NO:17);
xiii. comprises or consists of the amino acid sequence QAQQSY (SEQ ID NO:18);
xiv. comprises or consists of the amino acid sequence QAQQSW (SEQ ID NO:19);
xv. comprises or consists of the amino acid sequence QAQQSF (SEQ ID NO:20);
xvi. comprises or consists of the amino acid sequence LSSTQAQ (SEQ ID NO:21);
xvii. comprises or consists of the amino acid sequence SSTQAQQ (SEQ ID NO:22);
xviii. comprises or consists of the amino acid sequence STQAQQS (SEQ ID NO:23);
xix. comprises or consists of the amino acid sequence TQAQQSY (SEQ ID NO:24);
xx. comprises or consists of the amino acid sequence TQAQQSW (SEQ ID NO:25);
xxi. comprises or consists of the amino acid sequence TQAQQSF (SEQ ID NO:26);
xxii. comprises or consists of the amino acid sequence LSSTQAQQ (SEQ ID NO:27);
xxiii. comprises or consists of the amino acid sequence SSTQAQQS (SEQ ID NO:28);
xxiv. comprises or consists of the amino acid sequence STQAQQSY (SEQ ID NO:29);
xxv. comprises or consists of the amino acid sequence STQAQQSW (SEQ ID NO:30);
xxvi. comprises or consists of the amino acid sequence STQAQQSF (SEQ ID NO:31);
xxvii. comprises or consists of the amino acid sequence SSTQAQQSW (SEQ ID NO:32);
xxviii. comprises or consists of the amino acid sequence SSTQAQQSF (SEQ ID NO:33);
xxix. consists of the amino acid sequence LSSTQAQQS (SEQ ID NO:2) and an additional aromatic amino acid at the C-terminus;
xxx. consists of the amino acid sequence SSTQAQQSY (SEQ ID NO:3) and an additional aromatic amino acid at the C-terminus;
xxxi. consists of the amino acid sequence LSSTQAQQSY (SEQ ID NO:1) and an additional aromatic amino acid at the C-terminus;
xxxii. consists of the amino acid sequence LSSTQAQQSW (SEQ ID NO:6) and an additional aromatic amino acid at the C-terminus; or
xxxiii. consists of the amino acid sequence LSSTQAQQSF (SEQ ID NO:7) and an additional aromatic amino acid at the C-terminus.

4. A pharmaceutical composition comprising a compound that is a conjugate or a salt thereof, the conjugate comprising:
a. a peptide moiety whose amino acid sequence:
i. consists of
(I) 5 to 10 consecutive amino acids from the amino acid sequence LSSTQAQQSX₁ (SEQ ID NO:34), where X₁ is Y, W, or F; and
(II) optionally, an additional aromatic amino acid at the C-terminus of said 5 to 10 consecutive amino acids; and
iii. does not consist of the amino acid sequence LSSTQAQQSY (SEQ ID NO:1), LSSTQAQQSW (SEQ ID NO:6), or LSSTQAQQSF (SEQ ID NO:7);
attached to
b. one or more conjugate moieties.

5. The pharmaceutical composition of claim 4, wherein the peptide moiety consists of:
a. 6 to 10 consecutive amino acids from the amino acid sequence LSSTQAQQSX₁ (SEQ ID NO:34) and, optionally, an additional aromatic amino acid at the C-terminus of said 6 to 10 consecutive amino acids;
b. 7 to 10 consecutive amino acids from the amino acid sequence LSSTQAQQSX₁ (SEQ ID NO:34) and, optionally, an additional aromatic amino acid at the C-terminus of said 7 to 10 consecutive amino acids; or
c. 8 to 10 consecutive amino acids from the amino acid sequence LSSTQAQQSX₁ (SEQ ID NO:34) and, optionally, an additional aromatic amino acid at the C-terminus of said 8 to 10 consecutive amino acids, optionally wherein said 8 to 10 consecutive amino acids consists of amino acid sequence SSTQAQQS (SEQ ID NO:28).

6. The pharmaceutical composition of claim 4 or claim 5, wherein the peptide moiety is 5 amino acids in length, 6 amino acids in length, 7 amino acids in length, 8 amino acids in length, 9 amino acids in length, 10 amino acids in length, or 11 amino acids in length, optionally wherein the peptide moiety:
i. comprises or consists of the amino acid sequence LSSTQ (SEQ ID NO:4);
ii. comprises or consists of the amino acid sequence SSTQA (SEQ ID NO:8);
iii. comprises or consists of the amino acid sequence STQAQ (SEQ ID NO:9);
iv. comprises or consists of the amino acid sequence TQAQQ (SEQ ID NO:10);
v. comprises or consists of the amino acid sequence QAQQS (SEQ ID NO:11);
vi. comprises or consists of the amino acid sequence AQQSY (SEQ ID NO:5);
vii. comprises or consists of the amino acid sequence AQQSW (SEQ ID NO:12);
viii. comprises or consists of the amino acid sequence AQQSF (SEQ ID NO:13);
ix. comprises or consists of the amino acid sequence LSSTQA (SEQ ID NO:14);
x. comprises or consists of the amino acid sequence SSTQAQ (SEQ ID NO:15);
xi. comprises or consists of the amino acid sequence STQAQQ (SEQ ID NO:16);
xii. comprises or consists of the amino acid sequence TQAQQS (SEQ ID NO:17);
xiii. comprises or consists of the amino acid sequence QAQQSY (SEQ ID NO:18);
xiv. comprises or consists of the amino acid sequence QAQQSW (SEQ ID NO:19);
xv. comprises or consists of the amino acid sequence QAQQSF (SEQ ID NO:20);
xvi. comprises or consist of the amino acid sequence LSSTQAQ (SEQ ID NO:21);
xvii. comprises or consists of the amino acid sequence SSTQAQQ (SEQ ID NO:22);
xviii. comprises or consists of the amino acid sequence STQAQQS (SEQ ID NO:23);
xix. comprises or consists of the amino acid sequence TQAQQSY (SEQ ID NO:24);
xx. comprises or consists of the amino acid sequence TQAQQSW (SEQ ID NO:25);
xxi. comprises or consists of the amino acid sequence TQAQQSF (SEQ ID NO:26);
xxii. comprises or consists of the amino acid sequence LSSTQAQQ (SEQ ID NO:27);
xxiii. comprises or consists of the amino acid sequence SSTQAQQS (SEQ ID NO:28);
xxiv. comprises or consists of the amino acid sequence STQAQQSY (SEQ ID NO:29);
xxv. comprises or consists of the amino acid sequence STQAQQSW (SEQ ID NO:30);
xxvi. comprises or consists of the amino acid sequence STQAQQSF (SEQ ID NO:31);
xxvii. comprises or consists of the amino acid sequence LSSTQAQQS (SEQ ID NO:2);
xxviii. comprises or consists of the amino acid sequence SSTQAQQSY (SEQ ID NO:3);
xxix. comprises or consists of the amino acid sequence SSTQAQQSW (SEQ ID NO:32);
xxx. comprises or consists of the amino acid sequence SSTQAQQSF (SEQ ID NO:33);
xxxi. consists of the amino acid sequence LSSTQAQQSY (SEQ ID NO:1) and an additional aromatic amino acid at the C-terminus;
xxxii. consists of the amino acid sequence LSSTQAQQSW (SEQ ID NO:6) and an additional aromatic amino acid at the C-terminus; or
xxxiii. consists of the amino acid sequence LSSTQAQQSF (SEQ ID NO:7) and an additional aromatic amino acid at the C-terminus.

7. The pharmaceutical composition of any one of claims 4 to 6, wherein at least one of the one or more conjugate moieties comprises a polymer, an amino group, an acyl group, an alkyl group, a phosphate group, a lipid or a sugar.

8. The pharmaceutical composition of any one of claims 4 to 7 wherein the compound comprises:
a. a single conjugate moiety; or
b. more than one conjugate moiety.

9. The pharmaceutical composition of any one of claims 1 to 8, which is a salt, optionally wherein the salt is an acid addition salt or a base addition salt.

10. The pharmaceutical composition of any one of claims 1 to 9, wherein the compound is 0.1% to less than 100%, 1% to 99%, 1% to 90%, 5% to 80%, 10% to 75%, or 15% to 50% of the pharmaceutical composition by weight.

11. The pharmaceutical composition of any one of claims 1 to 10, wherein the pharmaceutical composition is formulated for administration to a subject at a fixed dose ranging from 0.1 mg to 50 g, 0.1 mg to 10 g, 0.1 mg to 3 g, 0.1 mg to 100 mg, 0.1 mg to 1 mg, 0.3 mg to 3 g, or 0.3 mg to 100 mg of the compound per day.

12. The pharmaceutical composition of any one of claims 1 to 11, which is an enteric coated solid pharmaceutical composition.

13. The pharmaceutical composition of any one of claims 1 to 12, which is a tablet or capsule.

14. The pharmaceutical composition of any one of claims 1 to 13, which comprises a binder, lubricant, disintegrating agent, coloring agent, flavoring agent, melting agent, or a combination thereof.

15. A pharmaceutical composition according to any one of claims 1 to 14 for use in a method for the treatment of (a) a mood disorder, which is optionally depression, bipolar disorder, or adjustment disorder, (b) an anxiety disorder, or (c) a disorder of diminished motivation, which is optionally apathy, abulia, or akinetic mutism.

## Patentansprüche

1. Pharmazeutische Zusammensetzung, umfassend eine Verbindung, die ein Peptid oder ein Salz davon ist, und einen oder mehrere pharmazeutisch verträgliche Träger, Verdünnungsmittel und/oder Exzipienten, wobei die Aminosäuresequenz des Peptids:
a. besteht aus:
i. 5 bis 10 aufeinanderfolgenden Aminosäuren aus der Aminosäuresequenz LSSTQAQQSX₁ (SEQ ID NO:34), wobei X₁ Y, W oder F ist; und
ii. optional einer zusätzlichen aromatischen Aminosäure am C-Terminus der 5 bis 10 aufeinanderfolgenden Aminosäuren; und
b. nicht besteht aus der Aminosäuresequenz LSSTQAQQSY (SEQ ID NO:1), LSSTQAQQS (SEQ ID NO:2), SSTQAQQSY (SEQ ID NO:3), LSSTQ (SEQ ID NO:4), AQQSY (SEQ ID NO:5), LSSTQAQQSW (SEQ ID NO:6), oder LSSTQAQQSF (SEQ ID NO:7).

2. Pharmazeutische Zusammensetzung gemäß Anspruch 1, wobei die Aminosäuresequenz des Peptids besteht aus:
a. 6 bis 10 aufeinanderfolgenden Aminosäuren aus der Aminosäuresequenz LSSTQAQQSX₁ (SEQ ID NO:34) und optional einer zusätzlichen aromatischen Aminosäure am C-Terminus der 6 bis 10 aufeinanderfolgenden Aminosäuren;
b. 7 bis 10 aufeinanderfolgenden Aminosäuren aus der Aminosäuresequenz LSSTQAQQSX₁ (SEQ ID NO:34) und optional einer zusätzlichen aromatischen Aminosäure am C-Terminus der 7 bis 10 aufeinanderfolgenden Aminosäuren; oder
c. 8 bis 10 aufeinanderfolgenden Aminosäuren aus der Aminosäuresequenz LSSTQAQQSX₁ (SEQ ID NO: 34) und optional einer zusätzlichen aromatischen Aminosäure am C-Terminus der 8 bis 10 aufeinanderfolgenden Aminosäuren, wobei die 8 bis 10 aufeinanderfolgenden Aminosäuren optional aus der Aminosäuresequenz SSTQAQQS (SEQ ID NO:28) bestehen.

3. Pharmazeutische Zusammensetzung gemäß Anspruch 1 oder Anspruch 2, wobei das Peptid 5 Aminosäuren lang ist, 6 Aminosäuren lang ist, 7 Aminosäuren lang ist, 8 Aminosäuren lang ist, 9 Aminosäuren lang ist, 10 Aminosäuren lang ist oder 11 Aminosäuren lang ist, optional wobei das Peptid:
i. die Aminosäuresequenz SSTQA (SEQ ID NO:8) umfasst oder aus dieser besteht;
ii. die Aminosäuresequenz STQAQ (SEQ ID NO:9) umfasst oder aus dieser besteht;
iii. die Aminosäuresequenz TQAQQ (SEQ ID NO:10) umfasst oder aus dieser besteht;
iv. die Aminosäuresequenz QAQQS (SEQ ID NO:11) umfasst oder aus dieser besteht;
v. die Aminosäuresequenz AQQSW (SEQ ID NO:12) umfasst oder aus dieser besteht;
vi. die Aminosäuresequenz AQQSF (SEQ ID NO:13) umfasst oder aus dieser besteht;
vii. aus der Aminosäuresequenz LSSTQ (SEQ ID NO:4) und einer zusätzlichen aromatischen Aminosäure am C-Terminus besteht;
viii. aus der Aminosäuresequenz AQQSY (SEQ ID NO:5) und einer zusätzlichen aromatischen Aminosäure am C-Terminus besteht;
ix. die Aminosäuresequenz LSSTQA (SEQ ID NO:14) umfasst oder aus dieser besteht;
x. die Aminosäuresequenz SSTQAQ (SEQ ID NO:15) umfasst oder aus dieser besteht;
xi. die Aminosäuresequenz STQAQQ (SEQ ID NO:16) umfasst oder aus dieser besteht;
xii. die Aminosäuresequenz TQAQQS (SEQ ID NO:17) umfasst oder aus dieser besteht;
xiii. die Aminosäuresequenz QAQQSY (SEQ ID NO:18) umfasst oder aus dieser besteht;
xiv. die Aminosäuresequenz QAQQSW (SEQ ID NO:19) umfasst oder aus dieser besteht;
xv. die Aminosäuresequenz QAQQSF (SEQ ID NO:20) umfasst oder aus dieser besteht;
xvi. die Aminosäuresequenz LSSTQAQ (SEQ ID NO:21) umfasst oder aus dieser besteht;
xvii. die Aminosäuresequenz SSTQAQQ (SEQ ID NO:22) umfasst oder aus dieser besteht;
xviii. die Aminosäuresequenz STQAQQS (SEQ ID NO:23) umfasst oder aus dieser besteht;
xix. die Aminosäuresequenz TQAQQSY (SEQ ID NO:24) umfasst oder aus dieser besteht;
xx. die Aminosäuresequenz TQAQQSW (SEQ ID NO:25) umfasst oder aus dieser besteht;
xxi. die Aminosäuresequenz TQAQQSF (SEQ ID NO:26) umfasst oder aus dieser besteht;
xxii. die Aminosäuresequenz LSSTQAQQ (SEQ ID NO:27) umfasst oder aus dieser besteht;
xxiii. die Aminosäuresequenz SSTQAQQS (SEQ ID NO:28) umfasst oder aus dieser besteht;
xxiv. die Aminosäuresequenz STQAQQSY (SEQ ID NO:29) umfasst oder aus dieser besteht;
xxv. die Aminosäuresequenz STQAQQSW (SEQ ID NO:30) umfasst oder aus dieser besteht;
xxvi. die Aminosäuresequenz STQAQQSF (SEQ ID NO:31) umfasst oder aus dieser besteht;
xxvii. die Aminosäuresequenz SSTQAQQSW (SEQ ID NO:32) umfasst oder aus dieser besteht;
xxviii. die Aminosäuresequenz SSTQAQQSF (SEQ ID NO:33) umfasst oder aus dieser besteht;
xxix. aus der Aminosäuresequenz LSSTQAQQS (SEQ ID NO:2) und einer zusätzlichen aromatischen Aminosäure am C-Terminus besteht;
xxx. aus der Aminosäuresequenz SSTQAQQSY (SEQ ID NO:3) und einer zusätzlichen aromatischen Aminosäure am C-Terminus besteht;
xxxi. aus der Aminosäuresequenz LSSTQAQQSY (SEQ ID NO:1) und einer zusätzlichen aromatischen Aminosäure am C-Terminus besteht;
xxxii. aus der Aminosäuresequenz LSSTQAQQSW (SEQ ID NO:6) und einer zusätzlichen aromatischen Aminosäure am C-Terminus besteht; oder
xxxiii. aus der Aminosäuresequenz LSSTQAQQSF (SEQ ID NO:7) und einer zusätzlichen aromatischen Aminosäure am C-Terminus besteht.

4. Pharmazeutische Zusammensetzung, umfassend eine Verbindung, die ein Konjugat oder ein Salz davon ist, wobei das Konjugat umfasst:
a. eine Peptidkomponente, deren Aminosäuresequenz:
i. besteht aus
(I) 5 bis 10 aufeinanderfolgenden Aminosäuren aus der Aminosäuresequenz LSSTQAQQSX₁ (SEQ ID NO:34), wobei X₁ Y, W oder F ist; und
(II) optional einer zusätzlichen aromatischen Aminosäure am C-Terminus der 5 bis 10 aufeinanderfolgenden Aminosäuren; und
iii. nicht besteht aus der Aminosäuresequenz LSSTQAQQSY (SEQ ID NO:1), LSSTQAQQSW (SEQ ID NO:6), oder LSSTQAQQSF (SEQ ID NO:7);
verbunden mit
b. einer oder mehreren konjugierten Komponenten.

5. Pharmazeutische Zusammensetzung gemäß Anspruch 4, wobei die Peptidkomponente besteht aus:
a. 6 bis 10 aufeinanderfolgenden Aminosäuren aus der Aminosäuresequenz LSSTQAQQSX₁ (SEQ ID NO:34) und optional einer zusätzlichen aromatischen Aminosäure am C-Terminus der 6 bis 10 aufeinanderfolgenden Aminosäuren;
b. 7 bis 10 aufeinanderfolgenden Aminosäuren aus der Aminosäuresequenz LSSTQAQQSX₁ (SEQ ID NO:34) und optional einer zusätzlichen aromatischen Aminosäure am C-Terminus der 7 bis 10 aufeinanderfolgenden Aminosäuren; oder
c. 8 bis 10 aufeinanderfolgenden Aminosäuren aus der Aminosäuresequenz LSSTQAQQSX₁ (SEQ ID NO:34) und optional einer zusätzlichen aromatischen Aminosäure am C-Terminus der 8 bis 10 aufeinanderfolgenden Aminosäuren, wobei die 8 bis 10 aufeinanderfolgenden Aminosäuren optional aus der Aminosäuresequenz SSTQAQQS (SEQ ID NO:28) bestehen.

6. Pharmazeutische Zusammensetzung gemäß Anspruch 4 oder Anspruch 5, wobei die Peptidkomponente 5 Aminosäuren lang ist, 6 Aminosäuren lang ist, 7 Aminosäuren lang ist, 8 Aminosäuren lang ist, 9 Aminosäuren lang ist, 10 Aminosäuren lang ist oder 11 Aminosäuren lang ist, optional wobei die Peptidkomponente:
i. die Aminosäuresequenz LSSTQ (SEQ ID NO:4) umfasst oder aus dieser besteht;
ii. die Aminosäuresequenz SSTQA (SEQ ID NO:8) umfasst oder aus dieser besteht;
iii. die Aminosäuresequenz STQAQ (SEQ ID NO:9) umfasst oder aus dieser besteht;
iv. die Aminosäuresequenz TQAQQ (SEQ ID NO:10) umfasst oder aus dieser besteht;
v. die Aminosäuresequenz QAQQS (SEQ ID NO:11) umfasst oder aus dieser besteht;
vi. die Aminosäuresequenz AQQSY (SEQ ID NO:5) umfasst oder aus dieser besteht;
vii. die Aminosäuresequenz AQQSW (SEQ ID NO:12) umfasst oder aus dieser besteht;
viii. die Aminosäuresequenz AQQSF (SEQ ID NO:13) umfasst oder aus dieser besteht;
ix. die Aminosäuresequenz LSSTQA (SEQ ID NO:14) umfasst oder aus dieser besteht;
x. die Aminosäuresequenz SSTQAQ (SEQ ID NO:15) umfasst oder aus dieser besteht;
xi. die Aminosäuresequenz STQAQQ (SEQ ID NO:16) umfasst oder aus dieser besteht;
xii. die Aminosäuresequenz TQAQQS (SEQ ID NO:17) umfasst oder aus dieser besteht;
xiii. die Aminosäuresequenz QAQQSY (SEQ ID NO:18) umfasst oder aus dieser besteht;
xiv. die Aminosäuresequenz QAQQSW (SEQ ID NO:19) umfasst oder aus dieser besteht;
xv. die Aminosäuresequenz QAQQSF (SEQ ID NO:20) umfasst oder aus dieser besteht;
xvi. die Aminosäuresequenz LSSTQAQ (SEQ ID NO:21) umfasst oder aus dieser besteht;
xvii. die Aminosäuresequenz SSTQAQQ (SEQ ID NO:22) umfasst oder aus dieser besteht;
xviii. die Aminosäuresequenz STQAQQS (SEQ ID NO:23) umfasst oder aus dieser besteht;
xix. die Aminosäuresequenz TQAQQSY (SEQ ID NO:24) umfasst oder aus dieser besteht;
xx. die Aminosäuresequenz TQAQQSW (SEQ ID NO:25) umfasst oder aus dieser besteht;
xxi. die Aminosäuresequenz TQAQQSF (SEQ ID NO:26) umfasst oder aus dieser besteht;
xxii. die Aminosäuresequenz LSSTQAQQ (SEQ ID NO:27) umfasst oder aus dieser besteht;
xxiii. die Aminosäuresequenz SSTQAQQS (SEQ ID NO:28) umfasst oder aus dieser besteht;
xxiv. die Aminosäuresequenz STQAQQSY (SEQ ID NO:29) umfasst oder aus dieser besteht;
xxv. die Aminosäuresequenz STQAQQSW (SEQ ID NO:30) umfasst oder aus dieser besteht;
xxvi. die Aminosäuresequenz STQAQQSF (SEQ ID NO:31) umfasst oder aus dieser besteht;
xxvii. die Aminosäuresequenz LSSTQAQQS (SEQ ID NO:2) umfasst oder aus dieser besteht;
xxviii. die Aminosäuresequenz SSTQAQQSY (SEQ ID NO:3) umfasst oder aus dieser besteht;
xxix. die Aminosäuresequenz SSTQAQQSW (SEQ ID NO:32) umfasst oder aus dieser besteht;
xxx. die Aminosäuresequenz SSTQAQQSF (SEQ ID NO:33) umfasst oder aus dieser besteht;
xxxi. aus der Aminosäuresequenz LSSTQAQQSY (SEQ ID NO:1) und einer zusätzlichen aromatischen Aminosäure am C-Terminus besteht;
xxxii. aus der Aminosäuresequenz LSSTQAQQSW (SEQ ID NO:6) und einer zusätzlichen aromatischen Aminosäure am C-Terminus besteht; oder
xxxiii. aus der Aminosäuresequenz LSSTQAQQSF (SEQ ID NO:7) und einer zusätzlichen aromatischen Aminosäure am C-Terminus besteht.

7. Pharmazeutische Zusammensetzung gemäß irgendeinem der Ansprüche 4 bis 6, wobei mindestens eine der einen oder mehreren konjugierten Komponenten ein Polymer, eine Aminogruppe, eine Acylgruppe, eine Alkylgruppe, eine Phosphatgruppe, ein Lipid oder einen Zucker umfasst.

8. Pharmazeutische Zusammensetzung gemäß irgendeinem der Ansprüche 4 bis 7, wobei die Verbindung umfasst:
a. eine einzelne konjugierte Komponente; oder
b. mehr als eine konjugierte Komponente.

9. Pharmazeutische Zusammensetzung gemäß irgendeinem der Ansprüche 1 bis 8, die ein Salz ist, optional wobei das Salz ein Säureadditionssalz oder ein Basenadditionssalz ist.

10. Pharmazeutische Zusammensetzung gemäß irgendeinem der Ansprüche 1 bis 9, wobei die Verbindung 0,1% bis weniger als 100%, 1% bis 99%, 1% bis 90%, 5% bis 80%, 10% bis 75% oder 15% bis 50% der pharmazeutischen Zusammensetzung nach Gewicht ausmacht.

11. Pharmazeutische Zusammensetzung gemäß irgendeinem der Ansprüche 1 bis 10, wobei die pharmazeutische Zusammensetzung zur Verabreichung an ein Subjekt in einer fixierten Dosis im Bereich von 0,1 mg bis 50 g, 0,1 mg bis 10 g, 0,1 mg bis 3 g, 0,1 mg bis 100 mg, 0,1 mg bis 1 mg, 0,3 mg bis 3 g oder 0,3 mg bis 100 mg der Verbindung pro Tag formuliert ist.

12. Pharmazeutische Zusammensetzung gemäß irgendeinem der Ansprüche 1 bis 11, bei der es sich um eine magensaftresistent beschichtete feste pharmazeutische Zusammensetzung handelt.

13. Pharmazeutische Zusammensetzung gemäß irgendeinem der Ansprüche 1 bis 12, die eine Tablette oder Kapsel ist.

14. Pharmazeutische Zusammensetzung gemäß irgendeinem der Ansprüche 1 bis 13, umfassend ein Bindemittel, Gleitmittel, Sprengmittel, Farbstoff, Geschmacksstoff, Schmelzmittel oder eine Kombination davon.

15. Pharmazeutische Zusammensetzung gemäß irgendeinem der Ansprüche 1 bis 14 zur Verwendung in einem Verfahren zur Behandlung von (a) einer Stimmungsstörung, die optional eine Depression, bipolare Störung oder Anpassungsstörung ist, (b) einer Angststörung, oder (c) einer Störung mit verminderter Motivation, die optional Apathie, Abulie oder akinetischer Mutismus ist.

## Revendications

1. Composition pharmaceutique comprenant un composé qui est un peptide ou un sel de celui-ci et un ou plusieurs supports, diluants et/ou excipients pharmaceutiquement acceptables, dans laquelle la séquence d'acides aminés du peptide :
a. consiste en :
i. 5 à 10 acides aminés consécutifs de la séquence d'acides aminés LSSTQAQQSX₁ (SEQ ID NO : 34), où X₁ est Y, W ou F ; et
ii. facultativement, un acide aminé aromatique supplémentaire à l'extrémité C-terminale desdits 5 à 10 acides aminés consécutifs ; et
b. ne consiste pas en la séquence d'acides aminés LSSTQAQQSY (SEQ ID NO : 1), LSSTQAQQS (SEQ ID NO : 2), SSTQAQQSY (SEQ ID NO : 3), LSSTQ (SEQ ID NO : 4), AQQSY (SEQ ID NO : 5), LSSTQAQQSW (SEQ ID NO : 6), ou LSSTQAQQSF (SEQ ID NO : 7).

2. Composition pharmaceutique selon la revendication 1, dans laquelle la séquence d'acides aminés du peptide consiste en :
a. 6 à 10 acides aminés consécutifs de la séquence d'acides aminés LSSTQAQQSX₁ (SEQ ID NO : 34) et, facultativement, un acide aminé aromatique supplémentaire à l'extrémité C-terminale desdits 6 à 10 acides aminés consécutifs ;
b. 7 à 10 acides aminés consécutifs de la séquence d'acides aminés LSSTQAQQSX₁ (SEQ ID NO : 34) et, facultativement, un acide aminé aromatique supplémentaire à l'extrémité C-terminale desdits 7 à 10 acides aminés consécutifs ; ou
c. 8 à 10 acides aminés consécutifs de la séquence d'acides aminés LSSTQAQQSX₁ (SEQ ID NO : 34) et, facultativement, un acide aminé aromatique supplémentaire à l'extrémité C-terminale desdits 8 à 10 acides aminés consécutifs, facultativement dans laquelle lesdits 8 à 10 acides aminés consécutifs consistent en la séquence d'acides aminés SSTQAQQS (SEQ ID NO : 28).

3. Composition pharmaceutique selon la revendication 1 ou la revendication 2, dans laquelle le peptide est de 5 acides aminés de longueur, 6 acides aminés de longueur, 7 acides aminés de longueur, 8 acides aminés de longueur, 9 acides aminés de longueur, 10 acides aminés de longueur, ou 11 acides aminés de longueur, facultativement dans laquelle le peptide :
i. comprend ou consiste en la séquence d'acides aminés SSTQA (SEQ ID NO: 8) ;
ii. comprend ou consiste en la séquence d'acides aminés STQAQ (SEQ ID NO: 9) ;
iii. comprend ou consiste en la séquence d'acides aminés TQAQQ (SEQ ID NO : 10) ;
iv. comprend ou consiste en la séquence d'acides aminés QAQQS (SEQ ID NO : 11) ;
v. comprend ou consiste en la séquence d'acides aminés AQQSW (SEQ ID NO : 12) ;
vi. comprend ou consiste en la séquence d'acides aminés AQQSF (SEQ ID NO : 13) ;
vii. consiste en la séquence d'acides aminés LSSTQ (SEQ ID NO : 4) et un acide aminé aromatique supplémentaire à l'extrémité C-terminale ;
viii. consiste en la séquence d'acides aminés AQQSY (SEQ ID NO : 5) et un acide aminé aromatique supplémentaire à l'extrémité C-terminale ;
ix. comprend ou consiste en la séquence d'acides aminés LSSTQA (SEQ ID NO : 14) ;
x. comprend ou consiste en la séquence d'acides aminés SSTQAQ (SEQ ID NO : 15) ;
xi. comprend ou consiste en la séquence d'acides aminés STQAQQ (SEQ ID NO : 16) ;
xii. comprend ou consiste en la séquence d'acides aminés TQAQQS (SEQ ID NO : 17) ;
xiii. comprend ou consiste en la séquence d'acides aminés QAQQSY (SEQ ID NO : 18) ;
xiv. comprend ou consiste en la séquence d'acides aminés QAQQSW (SEQ ID NO : 19) ;
xv. comprend ou consiste en la séquence d'acides aminés QAQQSF (SEQ ID NO : 20) ;
xvi. comprend ou consiste en la séquence d'acides aminés LSSTQAQ (SEQ ID NO : 21) ;
xvii. comprend ou consiste en la séquence d'acides aminés SSTQAQQ (SEQ ID NO : 22) ;
xviii. comprend ou consiste en la séquence d'acides aminés STQAQQS (SEQ ID NO : 23) ;
xix. comprend ou consiste en la séquence d'acides aminés TQAQQSY (SEQ ID NO : 24) ;
xx. comprend ou consiste en la séquence d'acides aminés TQAQQSW (SEQ ID NO : 25) ;
xxi. comprend ou consiste en la séquence d'acides aminés TQAQQSF (SEQ ID NO : 26) ;
xxii. comprend ou consiste en la séquence d'acides aminés LSSTQAQQ (SEQ ID NO : 27) ;
xxiii. comprend ou consiste en la séquence d'acides aminés SSTQAQQS (SEQ ID NO : 28) ;
xxiv. comprend ou consiste en la séquence d'acides aminés STQAQQSY (SEQ ID NO : 29) ;
xxv. comprend ou consiste en la séquence d'acides aminés STQAQQSW (SEQ ID NO : 30) ;
xxvi. comprend ou consiste en la séquence d'acides aminés STQAQQSF (SEQ ID NO : 31) ;
xxvii. comprend ou consiste en la séquence d'acides aminés SSTQAQQSW (SEQ ID NO : 32) ;
xxviii. comprend ou consiste en la séquence d'acides aminés SSTQAQQSF (SEQ ID NO : 33) ;
xxix. consiste en la séquence d'acides aminés LSSTQAQQS (SEQ ID NO : 2) et un acide aminé aromatique supplémentaire à l'extrémité C-terminale ;
xxx. consiste en la séquence d'acides aminés SSTQAQQSY (SEQ ID NO : 3) et un acide aminé aromatique supplémentaire à l'extrémité C-terminale ;
xxxi. consiste en la séquence d'acides aminés LSSTQAQQSY (SEQ ID NO : 1) et un acide aminé aromatique supplémentaire à l'extrémité C-terminale ;
xxxii. consiste en la séquence d'acides aminés LSSTQAQQSW (SEQ ID NO : 6) et un acide aminé aromatique supplémentaire à l'extrémité C-terminale ; ou
xxxiii. consiste en la séquence d'acides aminés LSSTQAQQSF (SEQ ID NO : 7) et un acide aminé aromatique supplémentaire à l'extrémité C-terminale.

4. Composition pharmaceutique comprenant un composé qui est un conjugué ou un sel de celui-ci, le conjugué comprenant :
a. une fraction peptidique dont la séquence d'acides aminés :
i. consiste en
(I) 5 à 10 acides aminés consécutifs de la séquence d'acides aminés LSSTQAQQSX₁ (SEQ ID NO : 34), où X₁ est Y, W, ou F ; et
(II) facultativement, un acide aminé aromatique supplémentaire à l'extrémité C-terminale desdits 5 à 10 acides aminés consécutifs ; et
iii. ne consiste pas en la séquence d'acides aminés LSSTQAQQSY (SEQ ID NO: 1), LSSTQAQQSW (SEQ ID NO: 6), ou LSSTQAQQSF (SEQ ID NO : 7) ;
attachée à
b. une ou plusieurs fractions conjuguées.

5. Composition pharmaceutique selon la revendication 4, dans laquelle la fraction peptidique consiste en :
a. 6 à 10 acides aminés consécutifs de la séquence d'acides aminés LSSTQAQQSX₁ (SEQ ID NO : 34) et, facultativement, un acide aminé aromatique supplémentaire à l'extrémité C-terminale desdits 6 à 10 acides aminés consécutifs ;
b. 7 à 10 acides aminés consécutifs de la séquence d'acides aminés LSSTQAQQSX₁ (SEQ ID NO : 34) et, facultativement, un acide aminé aromatique supplémentaire à l'extrémité C-terminale desdits 7 à 10 acides aminés consécutifs ; ou
c. 8 à 10 acides aminés consécutifs de la séquence d'acides aminés LSSTQAQQSX₁ (SEQ ID NO : 34) et, facultativement, un acide aminé aromatique supplémentaire à l'extrémité C-terminale desdits 8 à 10 acides aminés consécutifs, facultativement dans laquelle lesdits 8 à 10 acides aminés consécutifs consistent en une séquence d'acides aminés SSTQAQQS (SEQ ID NO : 28).

6. Composition pharmaceutique selon la revendication 4 ou la revendication 5, dans laquelle la fraction peptidique est de 5 acides aminés de longueur, 6 acides aminés de longueur, 7 acides aminés de longueur, 8 acides aminés de longueur, 9 acides aminés de longueur, 10 acides aminés de longueur, ou 11 acides aminés de longueur, facultativement dans laquelle la fraction peptidique :
i. comprend ou consiste en la séquence d'acides aminés LSSTQ (SEQ ID NO : 4) ;
ii. comprend ou consiste en la séquence d'acides aminés SSTQA (SEQ ID NO : 8) ;
iii. comprend ou consiste en la séquence d'acides aminés STQAQ (SEQ ID NO : 9) ;
iv. comprend ou consiste en la séquence d'acides aminés TQAQQ (SEQ ID NO : 10) ;
v. comprend ou consiste en la séquence d'acides aminés QAQQS (SEQ ID NO : 11) ;
vi. comprend ou consiste en la séquence d'acides aminés AQQSY (SEQ ID NO : 5) ;
vii. comprend ou consiste en la séquence d'acides aminés AQQSW (SEQ ID NO : 12) ;
viii. comprend ou consiste en la séquence d'acides aminés AQQSF (SEQ ID NO : 13) ;
ix. comprend ou consiste en la séquence d'acides aminés LSSTQA (SEQ ID NO : 14) ;
x. comprend ou consiste en la séquence d'acides aminés SSTQAQ (SEQ ID NO : 15) ;
xi. comprend ou consiste en la séquence d'acides aminés STQAQQ (SEQ ID NO : 16) ;
xii. comprend ou consiste en la séquence d'acides aminés TQAQQS (SEQ ID NO : 17) ;
xiii. comprend ou consiste en la séquence d'acides aminés QAQQSY (SEQ ID NO : 18) ;
xiv. comprend ou consiste en la séquence d'acides aminés QAQQSW (SEQ ID NO : 19) ;
xv. comprend ou consiste en la séquence d'acides aminés QAQQSF (SEQ ID NO : 20) ;
xvi. comprend ou consiste en la séquence d'acides aminés LSSTQAQ (SEQ ID NO : 21) ;
xvii. comprend ou consiste en la séquence d'acides aminés SSTQAQQ (SEQ ID NO : 22) ;
xviii. comprend ou consiste en la séquence d'acides aminés STQAQQS (SEQ ID NO : 23) ;
xix. comprend ou consiste en la séquence d'acides aminés TQAQQSY (SEQ ID NO : 24) ;
xx. comprend ou consiste en la séquence d'acides aminés TQAQQSW (SEQ ID NO : 25) ;
xxi. comprend ou consiste en la séquence d'acides aminés TQAQQSF (SEQ ID NO : 26) ;
xxii. comprend ou consiste en la séquence d'acides aminés LSSTQAQQ (SEQ ID NO : 27) ;
xxiii. comprend ou consiste en la séquence d'acides aminés SSTQAQQS (SEQ ID NO : 28) ;
xxiv. comprend ou consiste en la séquence d'acides aminés STQAQQSY (SEQ ID NO : 29) ;
xxv. comprend ou consiste en la séquence d'acides aminés STQAQQSW (SEQ ID NO : 30) ;
xxvi. comprend ou consiste en la séquence d'acides aminés STQAQQSF (SEQ ID NO : 31) ;
xxvii. comprend ou consiste en la séquence d'acides aminés LSSTQAQQS (SEQ ID NO : 2) ;
xxviii. comprend ou consiste en la séquence d'acides aminés SSTQAQQSY (SEQ ID NO : 3) ;
xxix. comprend ou consiste en la séquence d'acides aminés SSTQAQQSW (SEQ ID NO : 32) ;
xxx. comprend ou consiste en la séquence d'acides aminés SSTQAQQSF (SEQ ID NO : 33) ;
xxxi. consiste en la séquence d'acides aminés LSSTQAQQSY (SEQ ID NO : 1) et un acide aminé aromatique supplémentaire à l'extrémité C-terminale ;
xxxii. consiste en la séquence d'acides aminés LSSTQAQQSW (SEQ ID NO : 6) et un acide aminé aromatique supplémentaire à l'extrémité C-terminale ; ou
xxxiii. consiste en la séquence d'acides aminés LSSTQAQQSF (SEQ ID NO : 7) et un acide aminé aromatique supplémentaire à l'extrémité C-terminale.

7. Composition pharmaceutique selon l'une quelconque des revendications 4 à 6, dans laquelle au moins une des une ou plusieurs fractions conjuguées comprend un polymère, un groupe aminé, un groupe acyle, un groupe alkyle, un groupe phosphate, un lipide ou un sucre.

8. Composition pharmaceutique selon l'une quelconque des revendications 4 à 7, dans laquelle le composé comprend :
a. une fraction conjuguée unique ; ou
b. plus d'une fraction conjuguée.

9. Composition pharmaceutique selon l'une quelconque des revendications 1 à 8, qui est un sel, facultativement dans laquelle le sel est un sel d'addition d'acide ou un sel d'addition de base.

10. Composition pharmaceutique selon l'une quelconque des revendications 1 à 9, dans laquelle le composé est de 0,1 % à moins de 100 %, 1 % à 99 %, 1 % à 90 %, 5 % à 80 %, 10 % à 75 % ou 15 % à 50 % de la composition pharmaceutique en poids.

11. Composition pharmaceutique selon l'une quelconque des revendications 1 à 10, dans laquelle la composition pharmaceutique est formulée pour être administrée à un sujet à une dose fixe allant de 0,1 mg à 50 g, 0,1 mg à 10 g, 0,1 mg à 3 g, 0,1 mg à 100 mg, 0,1 mg à 1 mg, 0,3 mg à 3 g, ou 0,3 mg à 100 mg du composé par jour.

12. Composition pharmaceutique selon l'une quelconque des revendications 1 à 11, qui est une composition pharmaceutique solide à enrobage entérique.

13. Composition pharmaceutique selon l'une quelconque des revendications 1 à 12, qui est un comprimé ou une pilule.

14. Composition pharmaceutique selon l'une quelconque des revendications 1 à 13, qui comprend un liant, un lubrifiant, un agent de désintégration, un agent colorant, un agent aromatisant, un agent de fusion, ou une combinaison de ceux-ci.

15. Composition pharmaceutique selon l'une quelconque des revendications 1 à 14, pour utilisation dans un procédé de traitement (a) d'un trouble de l'humeur, qui est facultativement une dépression, un trouble bipolaire, ou un trouble de l'adaptation, (b) d'un trouble anxieux, ou (c) d'un trouble de la motivation diminuée, qui est facultativement une apathie, une aboulie, ou un mutisme akinétique.
